# EUROPÄISCHE PATENTSCHRIFT

(11) **EP 1 812 565 B1**
(45) Veröffentlichungstag und Bekanntmachung des Hinweises auf die Patenterteilung: **27.02.2013**
(21) Anmeldenummer: 05797743.1
(22) Anmeldetag: 14.10.2005
(51) Int. Cl.: C12N 9/16, C12N 15/55, C12N 15/63

(54) **Polypeptid mit Phytaseaktivität und dieses codierende Nukleotidsequenz**
Polypeptide having a phytase activity and nucleotide sequence coding therefor
Polypeptide presentant une activité de phytase et séquence nucléotidique codant ce polypeptide

(30) Priorität: 15.10.2004 DE 102004050410
(43) Veröffentlichungstag der Anmeldung: 01.08.2007
(73) Patentinhaber: AB Enzymes GmbH, 64293 Darmstadt (DE)
(72) Erfinder: NGUYEN, Khanh Quoc, 64385 Reichelsheim (DE); WINTER, Bruno, 70188 Stuttgart (DE)
(74) Vertreter: Hiebl, Inge Elisabeth
(86) Internationale Anmeldenummer: PCT/EP2005/011108
(87) Internationale Veröffentlichungsnummer: WO 2006/042719

(56) Entgegenhaltungen:
- WO-A-01/36607
- WO-A-01/90333
- WO-A-2004/015084

## Beschreibung

Die Erfindung betrifft ein rekombinantes DNA Molekül, das ein Polypeptid mit Phytaseaktivität codiert sowie das codierte Polypeptid als solches. Insbesondere betrifft die Erfindung ein rekombinantes DNA Molekül, das ein Polypeptid mit Phytaseaktivität codiert, wobei die DNA-Sequenz durch eine Variation der reifen Wildtyp-*E. coli*-Phytase erhalten worden ist, wobei definierte Aminosäurepositionen im Vergleich zur Wildtypsequenz modifiziert sind. Die Erfindung betrifft ferner ein Verfahren zur Expression der rekombinanten Phytase sowie deren Verwendung in der Lebensmittel- und Futtermitteltechnologie.

Phytinsäure oder Myoinositol-1,2,3,4,5,6-hexakisdihydrogenphosphat (abgekürzt Myoinositol-Hexakisphosphat) ist die Hauptquelle von Inositol und die primäre Speicherform von Phosphat in Pflanzensamen. In den Samen von Hülsenfrüchten liegt etwa 70% des Phosphatgehalts als ein gemischtes Kalium-, Magnesium- und Calciumsalz der Phytinsäure vor. Samen, Getreidekörner und Hülsenfrüchte sind wichtige Komponenten von Lebens- und Futtermittelzubereitungen, insbesondere von Tierfutterpräparaten; aber auch in der menschlichen Nahrung gewinnen Getreide- und Hülsenfrüchte zunehmend an Bedeutung.

Die Phosphateinheiten der Phytinsäure binden als Komplex zweiwertige und dreiwertige Kationen wie Metallionen, d.h. ernährungsphysiologisch wichtige Ionen wie Calcium, Eisen, Zink und Magnesium sowie die Spurenelemente Mangan, Kupfer und Molybdän. Daneben bindet Phytinsäure auch zu einem gewissen Ausmaß Proteine durch elektrostatische Wechselwirkung.

Phytinsäure und ihre Salze, die Phytate, werden oft nicht metabolisiert, da sie aus dem Magen-Darm-Trakt nicht absorbiert werden, d.h. weder der darin enthaltene Phosphor noch die chelatierten Metallionen, noch die gebundenen Proteine sind ernährungsphysiologisch verfügbar.

Da Phosphor ein wesentliches Element für das Wachstum aller Organismen ist, müssen Lebensmittel und Futtermittel mit anorganischem Phosphat supplementiert werden. Sehr oft müssen auch die ernährungsphysiologisch essenziellen Ionen wie Eisen und Calcium supplementiert werden. Ferner wird der ernährungsphysiologische Wert jeder Diät vermindert, da Proteine durch Phytinsäure gebunden werden. Folglich wird Phytinsäure oft als Anti-Nährwertfaktor bezeichnet.

Weiterhin wird aufgrund fehlender Metabolisierung von Phytinsäure der Phosphor des Phytats über den Magen-Darm-Trakt der Tiere ausgeschieden, was zu einer unerwünschten Phosphatverunreinigung der Umwelt führt, in deren Folge es beispielsweise zur Eutrophierung von Gewässern und zu exzessivem Algenwachstum kommen kann.

Phytinsäure oder Phytate (diese Ausdrücke werden im Folgenden synonym verwendet außer anders angegeben) können durch Phytasen abgebaut werden. Phytinsäure enthaltende Pflanzensamen enthalten endogene Phytaseenzyme. Bei deren Aufnahme sind die Phytate in Lebensmitteln bzw. Futtermitteln theoretisch durch die endogenen Pflanzen-Phytasen, durch Phytasen aus der Darmflora und durch Phytasen aus der Darmschleimhaut hydrolisierbar. In der Praxis ist jedoch das Hydrolysepotenzial der endogenen Pflanzen-Phytasen und der im Darm vorkommenden Phytasen, sofern vorhanden, bei weitem nicht ausreichend, um signifikant die Bioverfügbarkeit des in den Phytaten gebundenen Phosphors zu gewährleisten. Daher werden Lebens- bzw. Futtermitteln häufig exogene Phytasen zugesetzt.

Phytasen können durch Pflanzen sowie durch Mikroorganismen produziert werden. Unter den Mikroorganismen sind Phytase produzierende Bakterien sowie Phytase produzierende Pilze und Hefen bekannt.

Die natürlich vorkommenden Phytase-Produzenten haben jedoch den Nachteil, dass Phytase nur in bestimmten Mengen und mit definierten Eigenschaften gebildet wird. Wie vorstehend dargelegt besteht jedoch ein erhöhter Bedarf an Phytase insbesondere für die Lebensmittel- und Futtermittelindustrie.

Der vorliegenden Erfindung liegt somit die Aufgabe zu Grunde, ein Polypeptid mit Phytaseaktivität bereit zu stellen, das ökonomisch produziert werden kann. Insbesondere soll die Phytase kostengünstig produziert werden können. Die Phytase soll ferner die wesentlichen Eigenschaften der natürlichen *E*. *coli-*Wildtyp-Phytase beibehalten, sich jedoch durch eine erhöhte Aktivität im Kulturüberstand bzw. eine verbesserte Sekretierbarkeit auszeichnen. Zu den wesentlichen Eigenschaften der natürlichen Wildtyp-Phytase zählen insbesondere die Fähigkeit, die Verfügbarkeit von Phosphat in vivo und in vitro zu verbessern, sowie die Eignung als Backhilfsmittel.

Der vorliegenden Erfindung liegt ferner die Aufgabe zu Grunde, ein Gen für ein Polypeptid mit Phytaseaktivität bereit zu stellen, das bei Expression in einer Wirtszelle erhöhte Aktivität des so codierten Proteins im Kulturüberstand bzw. eine erhöhte Sekretion des Polypeptids zur Folge hat. Das Polypeptid soll ökonomisch und kostengünstig zu produzieren sein. Insbesondere soll die Expression des Polypeptids zu höheren Ausbeuten in eukaryotischen Mikroorganismen verglichen mit der Expression der Wildtyp-Phytase führen. Bereitgestellt werden sollen ferner die das Polypeptid codierenden DNA-Sequenzen, entsprechende DNA-Konstrukte und Vektoren sowie eine Quelle für das rekombinante Enzym, die für die kommerzielle Verwendung für Nahrungs- und Futtermittel und in industriellen Prozessen geeignet ist und das erfindungsgemäße Enzym enthaltende Zusammensetzungen.

Es wurde nun überraschenderweise gefunden, dass eine Mutation im Bereich von einschließlich Aminosäure 189 bis einschließlich 211 und/oder von Aminosäure einschließlich Position 137 bis einschließlich 152 der Wildtyp-Phytase von *E*. *coli* zu einer erhöhten Aktivität im gesamten Kulturüberstand durch das Protein Phytase führt ohne die günstigen Wirkungen und wesentlichen Eigenschaften der Wildtyp-*E. coli*-Phytase zu beeinträchtigen.

Mehrere Phytasen aus *E*. *coli* sind in der Literatur beschrieben, z. B. in Dassa et al., 1990, J. Bacteriol. 172:5497-5500 (Accession-Nr. M58708). Genetisch modifizierte Mutanten der *E. coli*-Phytase wurden ebenfalls bereits beschrieben, die zu einer erhöhten Thermostabilität und/oder höheren spezifischen Aktivitäten führten (Rodriguez et al., 2000, Arch. Biochem. Biophys., 382:105-112, Lanahan et al., 2003, US-Patentanmeldung 2003 0157646 A1, WO 01/90333). Eine ortspezifische Mutagenese von *Escherichia coli*-Phytase mit verbesserten enzymatischen Eigenschaften ist ferner in der WO 01/36607 beschrieben. Die hier an Position 200 beschriebene Val-Tyr-Mutante entspricht jedoch nicht der erfindungsgemäßen Mutation an Position 200, da gemäß der WO 01/36607 eine andere Zählweise der Sequenz verwendet wurde, d.h. es wurde die Leadersequenz in die Zählung einbezogen. Die Position 222 in der Sequenz gemäß dieser Druckschrift (WO 01/36607) entspricht somit der Position 200 der erfindungsgemäßen Zählung. Weitere Proteine mit Phytaseaktivität sind offenbart in WO 99/08539, WO 01/90333, WO 02/095003, WO 03/038035, WO 03/038111, WO 04/015084 und WO 00/71728.

Ferner beschreibt die Druckschrift Garrett et al.; Applied Environ. Microbiol., 2004, 70(5), 3041-3046 Mutanten der *E. coli*-Phytase mit einer erhöhten thermischen und gastrointestinalen Stabilität.

Der Stand der Technik enthält jedoch keine Beschreibung von Mutationen in der *E. coli*-Phytase, die zu einer erhöhten Produktion eines prokaryotischen Enzyms bei Expression durch einen eukaryotischen Mikroorganismus führen. Weiterhin enthält der Stand der Technik keine Hinweise, durch eine Variation und insbesondere eine Mutation der *E. coli*-Phytase eine erhöhte Aktivität im Kulturüberstand durch das so produzierte Enzym zu erzielen. Insbesondere enthält der Stand der Technik keinen Hinweis auf die funktionelle Bedeutung der Positionen 189 bis 211 und/oder der Positionen 137 bis 152 der Wildtyp-*E*. *coli*-Phytasesequenz. Ganz besonders enthält der Stand der Technik keinen Hinweis auf eine funktionelle Bedeutung der Position 200 der Wildtyp-*E. coli-*Phytasesequenz.

Gelöst wird diese Aufgabe durch ein rekombinantes DNA-Molekül, das nach Expression in einer prokaryotischen oder eukaryotischen Wirtszelle ein Polypeptid mit Phytaseaktivität codiert, wobei das rekombinante DNA-Molekül eine DNA-Sequenz, ausgewählt aus a) DNA-Sequenzen, die durch Variationen der reifen Wildtyp-*E. coli*-Phytasesequenz erhalten worden sind, wobei die DNA-Sequenzen mindestens eine Mutation aufweisen, die ausgewählt ist aus der Gruppe Val 200 → Leu, Val 200 → Ile, Val 200 → Pro, Val 200 → Tyr, Leu 207 → Tyr und Leu → Phe, b) DNA-Sequenzen, die aufgrund der Degeneriertheit des genetischen Codes mit den Sequenzen gemäß a) verwandt sind, umfasst, wobei das rekombinante DNA-Molekül bei Expression in einer geeigneten Wirtszelle mit einer erhöhten Aktivität des so codierten Proteins im Kulturüberstand einhergeht.

Die Erfindung betrifft ferner ein Verfahren zur Herstellung eines Polypeptids mit Phytaseaktivität nach rekombinanten Techniken umfassend die Züchtung rekombinanter prokaryotischer und/oder eukaryotischer Wirtszellen, die eine erfindungsgemäße Nucleinsäuresequenz enthalten unter Bedingungen, die die Expression des Enzyms fördern sowie die anschließende Gewinnung des Enzyms. Weiterhin betrifft die Erfindung die Verwendung des Polypeptids mit Phytaseaktivität in herkömmlichen Verfahren z. B. in Verfahren, die Mineralien aus Phytatkomplexen in Pflanzenmaterialien entweder in vitro freisetzen, z. B. bei der Behandlung von Futtermitteln, oder dessen Verwendung beim Backen oder in vivo freisetzen, d.h. die Verabreichung des Polypeptids mit Phytaseaktivität an Tiere. Ferner betrifft die Erfindung die Verwendung der erfindungsgemäßen Polynucleotidsequenzen zur Herstellung von Sonden zum Auffinden ähnlicher Sequenzen, die entsprechende Enzyme codieren, in anderen Organismen sowie zur Transformation von Wirtszellen.

Die Anmeldung beschreibt weiterhin eine aus dem Gen von *Aspergillus niger* Phytase abgeleitete Signalsequenz.

Die beigefügten Figuren erläutern die Erfindung näher:
- Fig. 1:: SDS-PAGE von Kulturüberständen aus mit den Plasmiden pKDa2 und pKDa4 transformierten Stämmen. Die Bahnen 1 und 6 enthalten Markerproteine. Die genaue Beschreibung ist in Beispiel 6 und Tabelle 3 enthalten.

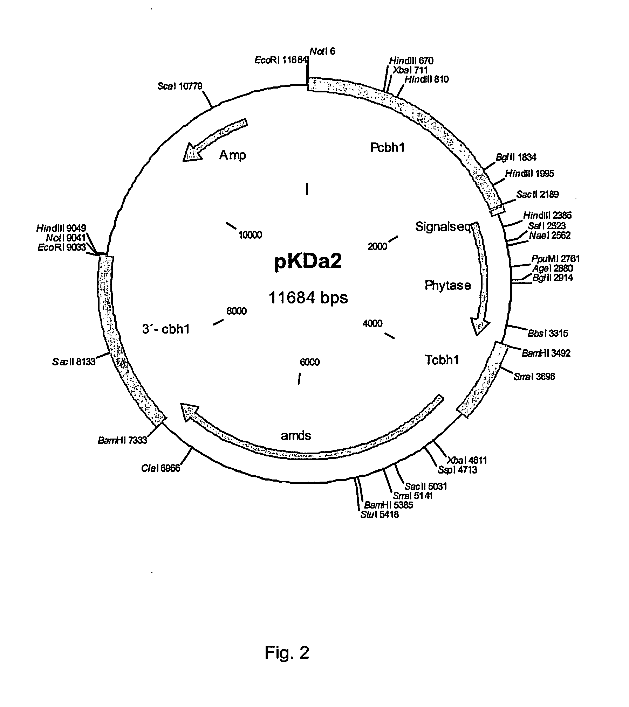

- Fig. 2:: Plasmidkarte von pKDa2 (Tyr²⁰⁰-Mutante)
- Fig. 3:: Plasmidkarte von pKDa4 (Wildtyp)

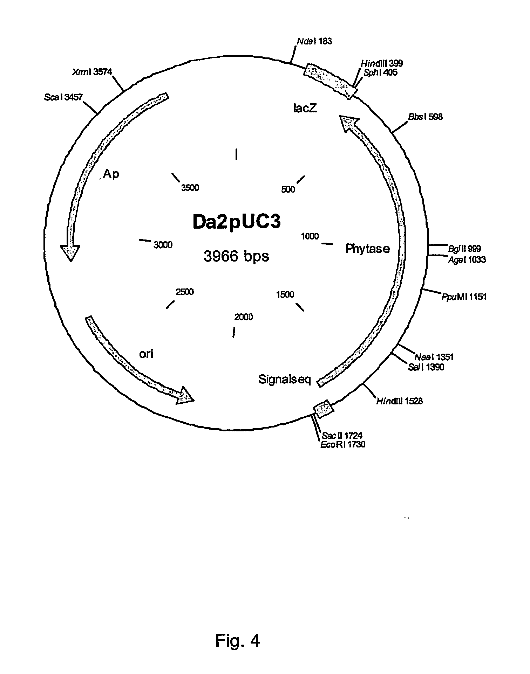

- Fig. 4:: Plasmidkarte von Da2pUC3

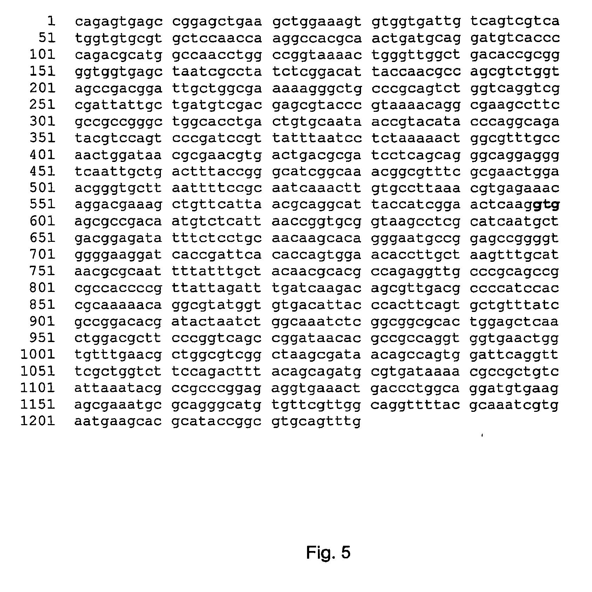

- Fig. 5:: DNA-Sequenz, die die *E. coli*-Wildtyp-Phytase codiert (CodonGebrauch von *E. coli*) (Dassa et al., 1990, J. Bacteriol. 172:5497 - 5500, Accession Nr.: M58704), reifes Protein

Das Plasmid Da2pUC3 wurde unter der Hinterlegungsnummer DSM 16396 am 7. Mai 2004 bei der Deutschen Sammlung von Mikroorganismen und Zellkulturen GmbH (DSMZ), Mascheroder Weg 1b, D-38124 Braunschweig gemäß den Bedingungen des Budapester Vertrags hinterlegt.

Überraschenderweise wurde gefunden, dass eine Aminosäuremutation im Bereich der Position 189 bis 211 (einschließlich der Grenzwerte) und/oder eine Aminosäuremutation im Bereich von Position 137 bis 152 (einschließlich der Grenzwerte) der Wildtyp-Phytasesequenz von *E*. *coli* mit einer deutlich erhöhten Aktivität im Kulturüberstand bei Expression in einer Wirtszelle einhergeht. Dabei handelt es sich um folgende Mutationen: Val 200 → Leu, Val 200 → Ile, Val 200 → Pro, Val 200 → Tyr, Leu 207 → Tyr und Leu 207 → Phe. Bevorzugte Mutationen sind Val 200 → Leu, Val 200 → Pro und Val 200 → Tyr. Besonders bevorzugt liegt eine Mutation an der Position Val 200 vor. Ohne durch die nachfolgende theoretische Erklärung gebunden zu werden, wird angenommen, dass durch die erfindungsgemäße Änderung Veränderungen in zwei räumlich benachbarten Strukturbereichen der Phytase, d.h. des β-Faltblatts und der α-Helix entstehen und diese Bereiche dadurch durch hydrophobe Interaktion so verändert werden, dass eine bessere Packungsdichte entsteht. Diese wiederum führt zu einer erhöhten Aktivität im Kulturüberstand bzw. Sekretion eines derart modifizierten Enzyms. Im Rahmen der vorstehenden Maßgabe, können in den genannten Bereichen beliebige Mutationen vorgenommen und kombiniert werden, solange ein mit einer entsprechenden DNA-Sequenz transformierter Mikroorganismus die codierte

Phytase unter Beibehalt der enzymatischen Aktivität und der weiteren physiologischen Eigenschaften der Wildtyp-*E. coli*-Phytase in erhöhten Mengen bzw. mit der Eigenschaft zu erhöhter Aktivität (Gesamtaktivität) im Kulturüberstand zu führen aus der Wirtszelle sezerniert. Bevorzugt beträgt die Verbesserung der Aktivität im Kulturüberstand bzw. Sekretionseffizienz einer Mutante verglichen mit der Wildtyp-*E. coli*-Phytase mindestens 10%, bevorzugter mindestens 20% bei Messung unter identischen Bedingungen. Die Bestimmung der enzymatischen Aktivität der Phytase sowie die Messung weiterer physiologischer Eigenschaften der Phytase können nach an sich bekannten Verfahren durchgeführt werden.

Die Erzielung einer mindestens 10%igen Steigerung der Aktivität im Kulturüberstand bzw. der Sekretionseffizienz der Phytase verglichen mit der Sekretionseffizienz der Wildtyp-Phytase war überraschend und hat nicht nahe gelegen. Insbesondere war überraschend, dass die Mutante Val 200 → Tyr mit einer 100%igen Steigerung der Sekretionseffizienz, verglichen mit der Wildtyp-Phytase einhergeht (vgl. Beispiel 4).

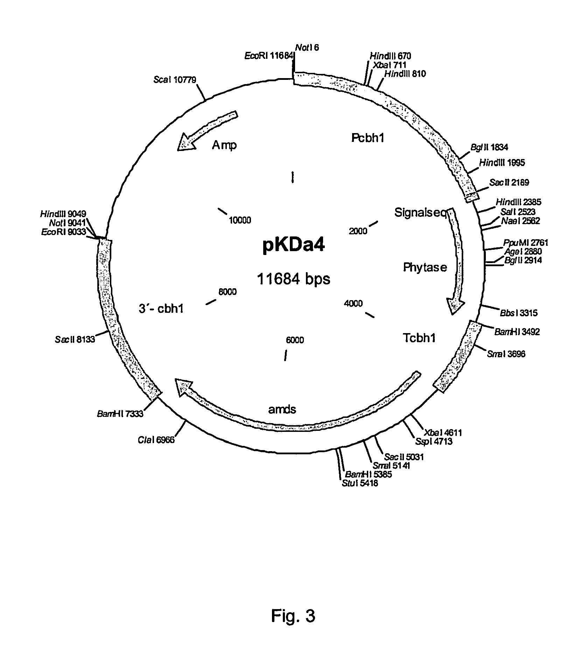

Im Stand der Technik sind Versuche beschrieben, die Eigenschaften von Phytase zu verbessern. So beschreibt die Druckschrift Archives Biochem. Biophys., 2000, 382(1), 105-112 auf Seite 109, Tabelle 3 die Aktivitäten der Phytase im Kulturüberstand bei Sekretion durch die Hefe *Pichia pastoris.* Die Wildtyp-*E*. *coli*-Phytase (die nicht genau sequenzidentisch mit der erfindungsgemäßen Sequenz pKDa4 ist) wurde nach 96 Stunden zu 117 Units ml⁻¹ sekretiert. Dabei ist zu bemerken, dass die Aktivitätsbestimmung gemäß dieser Druckschrift nicht identisch ist mit der Aktivitätsbestimmung gemäß Beispiel 4 der vorliegenden Erfindung. Gemäß dem Stand der Technik in der oben genannten Druckschrift wird das Substrat mit dem Enzym bei pH 2,5 inkubiert. Erfindungsgemäß erfolgt die Inkubation bei pH 5,0. Aus den Kurven auf Seite 109, Figur 3 der Druckschrift ergibt sich, dass 117 U ml⁻¹ bei pH 2,5 nur circa 60 U ml⁻¹ bei pH 5 entsprechen würden (52% Restaktivität). Somit beträgt die in dieser Druckschrift gemessene Enzymaktivität der sekretierten *E. coli*-Wildtyp-Phytase, codiert durch den *E. coli*-Wildtyp-Codongebrauch erheblich weniger als die durch den mit pKDa4 transformierten *T. reesei-*Stamm erhaltenen sekretierten Enzymaktivität von 158 bis 188 U ml⁻¹.

Die der erfindungsgemäß mutierten Phytasesequenz entsprechende DNA-Sequenz kann unter Verwendung beliebiger Codon usages realisiert werden, sofern durch den Codon usage die Sekretionshöhe nicht nachteilig beeinflusst wird. So kann beispielsweise die Codon usage des zur Expression verwendeten Mikroorganismus verwendet werden, es kann aber auch die *E. coli*-Codon usage bzw. eine Variante davon verwendet werden. Ferner kann die erfindungsgemäße mutierte *E. coli*-Phytasesequenz weitere Sequenzvariationen enthalten. Dabei können beliebige Variationen zusätzlich zu den vorstehend beschriebenen Mutationen vorgenommen werden, solange die Eigenschaft der Erzielung einer erhöhten Aktivität im Kulturüberstand bzw. Sekretionseffizienz nicht nachteilig beeinträchtigt wird und solange die enzymatische Aktivität und weitere wesentliche Eigenschaften der *E. coli*-Wildtyp-Phytase beibehalten werden.

Entsprechende Variationen sind einem Fachmann auf dem Gebiet der Technik der rekombinanten DNA gut bekannt und umfassen die vorstehenden Mutationen sowie die nachstehend dargelegten beispielhaften Variationen.

Erfindungsgemäß können Additions- und/oder Deletionsmoleküle des erfindungsgemäß modifizierten Polypeptids verwendet werden. So kann das erfindungsgemäß modifizierte Polypeptid mit Phytaseaktivität durch Anfügen weiterer Sequenzen am N-terminalen und/oder C-terminalen Ende verlängert werden. Dadurch können Hybridmoleküle hergestellt werden, die weitere vorteilhafte Eigenschaften besitzen. Beispielsweise können Fusionsproteine bzw. nativ in hohem Maße sekretierte Proteine angefügt werden, wodurch die Sekretionseffizienz weiter verbessert wird. Ferner können aktive Sequenzabschnitte anderer Enzyme hinzufügt werden, um Enzyme mit mehrfacher Spezifität zu erhalten. Ferner können polare oder unpolare Sequenzen angefügt werden, um die Löslichkeitseigenschaften bzw. die Membrangängigkeit des so erhaltenen Enzyms in gezielter Weise zu beeinflussen. Bevorzugt wird dabei das N-terminale Ende mit einer *Aspergillus*-Phytase oder einer sauren Phosphatase verknüpft. In gleicher Weise können Elongationen des C-Terminus der mutierten *E*. *coli-*Phytasesequenz vorgenommen werden. Dadurch können Phytasen mit einer geänderten Quarternärstruktur erhalten werden.

Erfindungsgemäß können auch Sequenzabschnitte des Polypeptids mit Phytaseaktivität deletiert werden, solange die Eigenschaft der erhöhten Sekretion bzw. Aktivität im Kulturüberstand unter Beibehaltung der Phytaseaktivität nicht beeinträchtigt wird.

Die Mutationen, Elongationen und Verkürzungen können in an sich bekannter Weise nach auf dem Fachgebiet gut bekannten Verfahren durchgeführt werden.

Die vorstehend in Betracht gezogenen Veränderungen des Polypeptids mit Phytaseaktivität entsprechen entsprechenden Mutationen bzw. Modifikationen des dazugehörigen DNA-Moleküls. Erfindungsgemäß werden dabei auch solche Sequenzen in Betracht gezogen, die unter relaxierten oder stringenten Bedingungen mit den erfindungsgemäßen Sequenzen hybridisieren. Ferner betrifft die Erfindung auch solche Sequenzen, die mit der beanspruchten Nucleotidsequenz bzw. den beanspruchten Teilen davon eine Homologie von mindestens 70%, bevorzugter mindestens 90% und insbesondere mindestens 95% aufweisen, solange die jeweiligen Sequenzen zur Erhöhung der Aktivität im Kulturüberstand bzw. der Sekretionseffizienz des durch sie codierten Polypeptids mit Phytaseaktivität führen. Bevorzugt beträgt die Homologie 70 bis zu 100 Prozent. Der Grad der Identität wird dabei bevorzugt so bestimmt, dass man die Anzahl der Reste der kürzeren Sequenz, die an dem Vergleich beteiligt ist und die ein "entsprechendes" Gegenstück in der anderen Sequenz besitzt, ermittelt. Für die Zwecke der vorliegenden Erfindung wird die Homologie dabei bevorzugt in üblicher Weise unter Verwendung der üblichen Algorithmen bestimmt. Erfindungsgemäß werden zum Vergleich nur die cDNAs der jeweiligen reifen Proteine herangezogen. Als homologe Sequenzen wurden erfindungsgemäß ähnliche, bevorzugt identische, Sequenzgegenstücke mit Hilfe von bekannten Computerprogrammen ermittelt. Ein Beispiel für ein derartiges Programm ist das Programm Clone Manager Suite, das den Programmteil Align Plus enthält und von Scientific & Educational Software, Durham, NC, USA vertrieben wird. Dabei wird ein Vergleich zweier wie vorstehend definierter DNA-Sequenzen durchgeführt, unter der Option local alignment entweder nach der Methode FastScan — MaxScore oder nach der Methode Needleman-Wunsch unter Beibehaltung der Defaultwerte. Speziell wurde erfindungsgemäß zur Berechnung der Homologie die Programmversion "Clone Manager 7 Align Plus 5" mit den Funktionen "Compare Two Sequences/Local Fast Scan-Max Score/Compare DNA sequences" angewendet. Dabei wurden die aus den folgenden Quellen zugänglichen Algorithmen verwendet: Hirschberg, D.S. 1975. A linear space algorithm for computing longest common subsequences. Commun Assoc Comput Mach 18:341-343; Myers, E.W. and W. Miller. 1988. Optimal alignments in linear space. CABIOS 4:1, 11-17; Chao, K-M, W.R. Pearson and W. Miller. 1992. A-ligning two sequences within a specified diagonal band. CABIOS 8:5, 481-487. Die Erfindung betrifft ferner auch DNA-Sequenzen, die aufgrund der Degeneriertheit des genetischen Codes mit den erfindungsgemäßen Sequenzen verwandt sind sowie allelische Varianten davon. Die Degeneriertheit des genetischen Codes kann sich dabei aufgrund natürlicher Degeneriertheit oder aufgrund eines speziell gewählten Codongebrauchs ergeben. Natürlich vorkommende allelische Varianten können unter Verwendung gut bekannter Techniken der Molekularbiologie wie z. B. der Polymerase-Kettenreaktion (PCR) und Hybridisierungstechniken identifiziert werden.

Eine DNA-Sequenz, die ein erfindungsgemäßes Polypeptid codiert, kann verwendet werden, um beliebige Wirtszellen wie beispielsweise Zellen von Pilzen, Hefen, Bakterien, Pflanzen oder Säugern zu transformieren. Derart transformierte Zellen zeichnen sich durch eine erhöhte Sekretion an Phytase aus. Das so produzierte Phytaseenzym führt auch zu einer effizienten Phosphatfreisetzung aus Myoinositolphosphaten.

Die Ausdrücke Protein, Peptid und Polypeptid sollen austauschbar verwendet werden. Ein Polypeptid oder Enzym mit Phytaseaktivität oder eine Phytase soll jedes Enzym bezeichnen, das die Freisetzung von anorganischem Phosphat aus verschiedenen Myoinositol-Phosphaten bewirken kann. Beispiele für solche Myoinositol-Phosphat(Phytase)-Substrate sind Phytinsäure und beliebige Salze davon, beispielsweise Natriumphytat oder Kaliumphytat oder gemischte Salze. Auch beliebige Positionsisomere der Di-, Tri-, Tetra- oder Pentaphosphate von Myoinositol können als Phytasesubstrat dienen. Die Phytaseaktivität kann unter Verwendung jedes beliebigen Assays, bei dem eines dieser Substrate verwendet wird, bestimmt werden. Eine erfindungsgemäße Phytase-Variante umfasst Polypeptidvarianten, die von einer speziellen Phytase durch Deletion oder Addition einer oder mehrerer Aminosäuren an das N-terminale und/oder C-terminale Ende des nativen Proteins, Deletion oder Addition einer oder mehrerer Aminosäuren an einer oder mehreren Stellen in dem nativen Protein, oder Substitution einer oder mehrerer Aminosäuren an einer oder mehreren Stellen in der Phytase abgeleitet sind. Die Erzeugung solcher Varianten ist allgemein auf dem Fachgebiet bekannt. Beispielsweise können Aminosäuresequenz-Varianten der Polypeptide durch Mutation in der DNA hergestellt werden. Verfahren zur Mutagenese und Nucleotidsequenz-Veränderung sind auf dem Fachgebiet gut bekannt (vgl. beispielsweise Kunkel, Proc. Natl. Acad. Sci. USA, 82:488 (1985), Kunkel et al., Methods in Enzymol., 154:367 (1987), US-Patentnr. 4,873,192, Walker und Gaastra, eds., Techniques in Molecular Biology, Mac Millan Publishing Company, New York (1983)). Hinweise über geeignete Aminosäuresubstitutionen, die die biologische Aktivität des Proteins von Interesse nicht beeinträchtigen, finden sich in dem Model von Dayhoff et al., Atlas of Protein Sequence and Structure, Natl. Biomed. Res. Found., Washington, D.C. (1978). Konservative Substitutionen wie der Austausch einer Aminosäure gegen eine andere mit ähnlichen Eigenschaften sind bevorzugt.

Die Erfindung betrifft auch isolierte oder im Wesentlichen gereinigte Nucleinsäure- oder Proteinzusammensetzungen. Dabei bezeichnet ein isoliertes und gereinigtes Polynucleotid/Polypeptid bzw. Segment davon ein Polynucleotid bzw. Polypeptid bzw. Segment davon, das isoliert aus seiner nativen Umgebung vorliegt. Ein isoliertes Polynucleinsäuresegment oder Polypeptid kann in gereinigter Form vorliegen oder kann in einer nicht nativen Umgebung vorliegen, wie beispielsweise in einer transgenen Wirtszelle. Beispielsweise ist ein isoliertes oder gereinigtes Polynucleotidsegment oder -Protein oder ein biologisch aktiver Teil davon im Wesentlichen frei von weiterem zellulärem Material oder Kulturmedium bei Produktion nach rekombinanten Techniken oder im Wesentlichen frei von chemischen Vorläufern oder anderen chemischen Verbindungen. Bevorzugt ist ein isoliertes Polynucleotid frei von Sequenzen (bevorzugt Proteincodierenden Sequenzen) die natürlicherweise die Nucleinsäure (d.h. Sequenzen, die an den 5'- und 3'-Enden der Nucleinsäure lokalisiert sind) in der genomischen DNA des Organismus aus dem die Nucleinsäure stammt, flankieren. Beispielsweise kann gemäß verschiedenen Ausführungsformen das isolierte Nucleinsäuremolekül weniger als etwa 5 kb, 4 kb, 3 kb, 2 kb, 1 kb, 0,5 kb oder 0,1 kb Nucleotidsequenzen enthalten, die natürlicherweise das Nucleotinsäuremolekül in der genomischen DNA der Zelle, aus der die Nucleinsäure abgeleitet ist, flankieren. Ein Protein, das im Wesentlichen frei von zellulärem Material ist, umfasst Zusammensetzungen von Protein oder Polypeptid mit weniger als etwa 70%, 50%, 30%, 20%, 10%, 5% (bezogen auf das Trockengewicht) von verunreinigendem Protein. Wenn das erfindungsgemäße Protein oder ein biologisch aktives Fragment davon rekombinant produziert wird, umfasst bevorzugt das Kulturmedium weniger als etwa 70%, 50%, 30%, 20%, 10% oder 5% (bezogen auf das Trockengewicht) der chemischen Vorläufer oder nichtproteinartigen chemischen Substanzen. Fragmente und Varianten der erfindungsgemäßen Nucleotidsequenzen oder Proteine oder Proteinsegmente, die dadurch codiert werden, sind ebenfalls erfindungsgemäß umfasst. Fragment bezeichnet einen Teil der Nucleotidsequenz oder einen Teil der Aminosäuresequenz und daher einen Teil des Polypeptids oder Proteins, welches davon codiert wird.

Die Erfindung betrifft auch Expressionskassetten, die zur Einschleusung des eine erfindungsgemäße Phytase codierenden offenen Leserasters in eine Wirtszelle verwendet werden können. Sie umfassen bevorzugt eine Transkriptionsstartregion, die mit dem offenen Leseraster verknüpft ist. Eine derartige Expressionskassette kann eine Vielzahl von Restriktionsspaltstellen zur Insertion des offenen Leserasters und/oder anderer DNAs, z. B. einer Transkriptions-Regulator-Region und/oder selektierbare Markergene enthalten. Die Transkriptionskassette umfasst in der 5'→3'-Richtung der Transkription eine Transkriptions- und Translationsstartregion, die DNA-Sequenz von Interesse und eine Transkriptions- und Translationsstopregion, die in einer mikrobiellen Zelle funktionell ist. Die Terminationsregion kann bezüglich der Transkriptioninitiationsregion nativ sein, kann bezüglich der DNA-Sequenz von Interesse nativ sein oder kann von einer beliebigen anderen Quelle abgeleitet sein.

Der Ausdruck "offenes Leseraster" (ORF) bezeichnet die Aminosäure-Sequenz, die zwischen den Translationsstart- und -Stop-Codons einer codierenden Sequenz codiert wird. Die Ausdrücke "Start-Codon" und "Stop-Codon" bezeichnen eine Einheit aus drei benachbarten Nucleotiden (Codons) in einer codierenden Sequenz, die den Kettenstart und -stop der Proteinsynthese (mRNA-Translation) spezifizieren.

"Funktionelle Verknüpfung" bezeichnet im Zusammenhang mit einer Nucleinsäure eine Verbindung als Teil des gleichen Nucleinsäuremoleküls in geeigneter Positionierung und Orientierung zum Transkriptionsstart des Promotors. DNA in funktioneller Verknüpfung an einen Promotor befindet sich unter der Transkriptions-Initiations-Regulation des Promotors. Codierende Sequenzen können funktionell mit der Regulatorsequenz in Sense- oder Antisense-Orientierung verknüpft sein. Bei Bezugnahme auf Polypeptide bedeutet funktionelle Verknüpfung die Verbindung als Teil desselben Polypeptids, d.h. über Peptidylbindungen.

Erfindungsgemäß können beliebige Promotoren verwendet werden. Promotor bezeichnet die Nucleotidsequenz üblicherweise stromaufwärts (5') bezüglich der codierenden Sequenz und kontrolliert die Expression der codierenden Sequenz, indem die Erkennung für die RNA-Polymerase und anderer Faktoren, die für die richtige Transkription erforderlich sind, bereit gestellt wird. Der erfindungsgemäß verwendete Promotor kann einen Minimalpromotor umfassen, d.h. eine kurze DNA-Sequenz aus einer TATA-Box und anderen Sequenzen, die die Transkriptionsstartstelle spezifizieren, an die Regulatorelemente zur Kontrolle der Expression angefügt sind.

Der erfindungsgemäße Promotor kann auch eine Nucleotidsequenz umfassen, die einen Minimalpromotor und Regulatorelemente umfasst, der die Expression einer codierenden Sequenz oder funktionellen RNA kontrollieren kann. Dieser Typ von Promotorsequenz besteht aus proximalen und distalen stromaufwärts gelegenen Elementen, wobei die zuletzt genannten Elemente oft als Enhancer bezeichnet werden. Folglich ist ein Enhancer eine DNA-Sequenz, die die Promotor-Aktivität stimulieren kann und kann ein dem Promotor innewohnendes Element oder ein insertiertes heterologes Element sein, um die Expressionshöhe oder Gewebspezifität eines Promotors zu verstärken. Er kann in beiden Orientierungen funktionieren und kann selbst bei stromaufwärtiger oder stromabwärtiger Platzierung von dem Promotor funktionieren. Sowohl Enhancer als auch andere stromaufwärts gelegene Promotor-Elemente binden sequenzspezifisch DNA-bindende Proteine, die ihre Wirkungen vermitteln. Promotoren können in ihrer Gesamtheit von einem nativen Gen abgeleitet sein oder können aus verschiedenen Elementen zusammengesetzt sein, die von verschiedenen natürlich vorkommenden Promotoren abgeleitet sind oder können sogar aus synthetischen DNA-Segmenten zusammengesetzt sein. Ein Promotor kann auch DNA-Sequenzen enthalten, die an der Bindung von Proteinfaktoren beteiligt sind, die die Effizienz der Transkriptionsinitiation als Antwort auf physiologische oder entwicklungsbedingte Bedingungen kontrollieren.

Promotorelemente, insbesondere TATA-Elemente, die inaktiv sind oder eine stark verminderte Promotor-Aktivität in Abwesenheit einer stromaufwärtigen Aktivierung besitzen, werden als Minimalpromotoren oder Kernpromotoren bezeichnet. In Gegenwart eines geeigneten Transkriptionfaktors bzw. geeigneter Transkriptionsfaktoren liegt die Funktion des Minimalpromotors in der Ermöglichung der Transkription. Ein Minimal- oder Kernpromotor besteht somit nur aus allen Grundelementen, die für die Transkriptionsinitiation notwendig sind, z. B. einer TATA-Box und/oder einem Initiator.

Die Erfindung betrifft auch die erfindungsgemäße DNA-enthaltende Vektoren. Diese Vektoren umfassen beliebige Plasmide, Cosmide, Phagen und andere Vektoren in doppelsträngiger oder einzelsträngiger, linearer oder zirkulärer Form, die gegebenenfalls selbst transmittierbar oder mobilisierbar sein können und die einen prokaryotischen oder eukaryotischen Wirt entweder durch Integration in das zelluläre Genom transformieren können oder die extrachromosomal vorliegen (z. B. autonom replizierende Plasmide mit einem Replikationsorigin).

Vektoren, Plasmide, Cosmide, künstliche Hefechromosomen (YACs), künstliche Bakterienchromosomen (BACs) und DNA-Segmente zur Verwendung zur Transformation von Zellen umfassen allgemein die erfindungsgemäße Phytase-codierende DNA sowie eine andere DNA, wie cDNA, ein Gen oder Gene, die in die Zellen eingeschleust werden sollen. Diese DNA-Konstrukte können weitere Strukturen wie Promotoren, Enhancer, Polylinker oder auch Regulatorgene, so weit erforderlich, umfassen. Eines der DNA-Segmente oder Gene, das bzw. die für die zelluläre Einschleusung ausgewählt wurde bzw. wurden, codiert/codieren zweckdienlicherweise ein Protein, das in den so erhaltenen transformierten (rekombinanten Zellen) exprimiert wird, was zu einem screenbaren oder selektierbaren Merkmal führt und/oder der transformierten Zelle einen verbesserten Phenotyp verleiht.

Die Konstruktion von Vektoren, die erfindungsgemäß verwendet werden können, ist einem Fachmann auf dem Gebiet angesichts der vorstehenden Offenbarung bekannt (vgl. z. B. Sambrook et al., Molecular Cloning: A Laboratory Manual (2. Aufl., Coldspring Harbor Laboratory Press, Plainview, N.Y. (1989)). Die erfindungsgemäße Expressionskassette kann ein oder mehrere Restriktionsschnittstellen enthalten, um das die Phytase-codierende Nucleotid unter die Regulation einer Regulatorsequenz zu stellen. Die Expressionskassette kann auch ein Terminationssignal in funktioneller Verknüpfung mit dem Polynucleotid sowie Regulatorsequenzen, die für die ordnungsgemäße Translation des Polynucleotids benötigt werden, enthalten. Die Expressionskassette, die das erfindungsgemäße Polynucleotid enthält, kann chimer sein, d.h. mindestens eine ihrer Komponenten ist bezogen auf mindestens eine der anderen Komponenten heterolog. Die Expression des Polynucleotids in der Expressionskassette kann unter der Kontrolle eines konstitutiven Promotors, eines induzierbaren Promotors, eines regulierten Promotors, eines viralen Promotors oder eines synthetischen Promotors stehen.

Die Vektoren können bereits Regulatorelemente enthalten, z. B. Promotoren, oder die erfindungsgemäßen DNA-Sequenzen können so manipuliert werden, dass sie solche Elemente enthalten. Geeignete Promotorelemente, die verwendet werden können, sind auf dem Fachgebiet bekannt und sind beispielsweise für *Trichoderma reesei* der cbh 1- oder der cbh-2-Promotor, für *Aspergillus ory*zae der amy-Promotor, für *Aspergillus niger* der xyl-, glaA-, alcA-, aphA-, tpiA-, gpdA-, sucl- und der pkiA-Promotor. Geeignete Promotorelemente, die zur Expression in Hefe verwendet werden können, sind auf dem Fachgebiet bekannt und sind beispielsweise der pho5-Promotor oder der gap-Promotor zur Expression in *Saccharomyces cerevisiae* und für *Pichia pastoris,* z. B. aoxl-Promotor oder der fmd-Promotor oder der mox-Promotor für *H. polymorpha.*

DNA, die zur Einschleusung in Zellen geeignet ist, kann neben der erfindungsgemäßen DNA auch DNA, die aus einer beliebigen Quelle abgeleitet wurde oder daraus isoliert ist, umfassen. Ein Beispiel für eine abgeleitete DNA ist eine DNA-Sequenz, die als nützliches Fragment in einem gegebenen Organismus identifiziert wurde und die dann in im Wesentlichen reiner Form chemisch synthetisiert wurde. Ein Beispiel für eine solche DNA ist eine geeignete DNA-Sequenz, die beispielsweise unter Verwendung von Restriktionsendonucleasen erhalten wurde, so dass sie weiter erfindungsgemäß manipuliert werden kann, beispielsweise amplifiziert werden kann. Eine derartige DNA wird üblicherweise als rekombinante DNA bezeichnet. Daher umfasst eine geeignete DNA vollständig synthetische DNA, semisynthetische DNA, aus biologischen Quellen isolierte DNA und von eingeschleuster RNA abgeleitete DNA. Im Allgemeinen ist die eingeschleuste DNA kein ursprünglicher Bestandteil des Genotyps der Empfänger-DNA, aber erfindungsgemäß kann auch ein Gen aus einem gegebenen Genotyp isoliert und eventuell verändert werden und anschließend können Mehrfachkopien des Gens in den gleichen Genotyp eingeschleust werden, z. B. um die Produktion eines gegebenen Genprodukts zu verstärken.

Die eingeschleuste DNA umfasst ohne Beschränkung DNA aus Genen, wie beispielsweise aus Bakterien, Hefen, Pilzen oder Viren. Die eingeschleuste DNA kann modifizierte oder synthetische Gene, Teile von Genen oder chimäre Gene einschließlich Genen aus dem gleichen oder einem unterschiedlichen Genotyp umfassen.

Die zur Transformation erfindungsgemäß verwendete DNA kann zirkulär oder linear, doppelsträngig oder einzelsträngig sein. Im Allgemeinen liegt die DNA in Form einer chimären DNA wie eine Plasmid-DNA vor, die auch codierende Regionen, die von Regulatorsequenzen flankiert sind, die die Expression der in der transformierten Zelle vorhandenen rekombinanten DNA unterstützen, enthalten. Beispielsweise kann die DNA selbst einen Promotor enthalten oder daraus bestehen, der in einer Zelle aktiv ist, der von einer Quelle, die sich von der Zelle unterscheidet, abgeleitet ist oder es kann ein Promotor verwendet werden, der bereits in der Zelle, d.h. der Transformationszielzelle, vorhanden ist.

Im Allgemeinen ist die eingeschleuste DNA relativ klein, weniger als etwa 30 kb, um die Empfindlichkeit gegenüber physikalischem, chemischem oder enzymatischem Abbau zu minimieren, der mit der Größe der DNA zunimmt.

Die Selektion eines geeigneten Expressionsvektors hängt von den Wirtszellen ab. Hefe- oder Pilzexpressionsvektoren können einen Replikationsorigin, einen geeigneten Promotor und Enhancer umfassen, und auch beliebige notwendige Ribosomenbindungsstellen, Polyadenylierungsstellen, Splice-Donor und -Akzeptor-Stellen, Transkriptionsterminationssequenzen und nicht-transkribierte 5'-flankierende Sequenzen umfassen.

Beispiele für geeignete Wirtszellen sind: Pilzzellen der Gattung *Aspergillus, Rhizopus, Trichoderma, Neurospora, Mucor, Penicillium,* etc., wie beispielsweise Hefen der Gattungen *Kluyveromyces, Saccharomyces, Schizosaccharomyces, Trichosporon, Schwanniomyces, Hansenula, Pichia* und dergleichen. Geeignete Wirtssysteme sind beispielsweise Pilze wie *Aspergilli,* z. B. *Aspergillus niger* (ATCC 9142) oder *Aspergillus ficuum* (NRLL 3135) oder *Trichoderma* (z. B. *Trichoderma reseei* QM6a) und Hefen wie *Saccharomyces,* z. B. *Saccharomyces cerevisiae* oder *Pichia,* wie z. B. *Pichia pastoris* oder *Hansenula,* z. B. *H. polymorpha* (DSMZ 70277). Derartige Mikroorganismen können von anerkannten Hinterlegungsstellen, z. B. der American Type Culture Collection (ATCC), dem Centraalbureau voor Schimmelcultures (CBS) oder der Deutschen Sammlung für Mikroorganismen und Zellkulturen GmbH (DSMZ) oder beliebigen anderen Hinterlegungsstellen erhalten werden.

Die Expressionskassette kann in der 5'-3'-Transkriptionsrichtung eine Transkriptions- und Translationstartregion des erfindungsgemäßen Polynucleotids und eine Transkriptions- und Terminationsregion, die in vivo oder in vitro funktionell ist, enthalten. Die Terminationsregion kann bezüglich der Transkriptionsinitiationsregion nativ sein oder kann bezüglich des Polynucleotids nativ oder anderer Herkunft sein. Die Regulatorsequenzen können stromaufwärts (5' nichtcodierende Sequenzen), innerhalb (Intron) oder stromabwärts (3' nichtcodierende Sequenzen) einer codierenden Sequenz lokalisiert sein und die Transkription, das RNA-Processing oder die Stabilität und/oder die Translation der assoziierten codierenden Sequenz beeinflussen. Regulatorsequenzen können ohne Beschränkung Enhancer, Promotoren, Repressorbindungsstellen, Translationsleadersequenzen, Introns oder Polyadenylierungsignalsequenzen umfassen. Sie können natürliche und synthetische Sequenzen sowie Sequenzen umfassen, die eine Kombination aus synthetischen und natürlichen Sequenzen sind.

Der erfindungsgemäß verwendete Vektor kann auch geeignete Sequenzen zur Amplifikation der Expression umfassen.

Beispiele für Promotoren, die erfindungsgemäß verwendet werden können, sind Promotoren, von denen bekannt ist, dass sie die Expression in den eukaryotischen Zellen kontrollieren. Beliebige Promotoren mit der Fähigkeit zur Expression in Fadenpilzen können verwendet werden. Beispiele sind ein Promotor, der stark durch Stärke oder Zellulose induziert wird, z. B. ein Promotor für Glucoamylase oder α-Amylase aus der Gattung *Aspergillus* oder Cellulase (Cellobiohydrolase) aus der Gattung *Trichoderma,* ein Promotor für Enzyme im glykolytischen Stoffwechselweg, wie beispielsweise Phosphoglyceratkinase (PGK) und Glycerinaldehyd-3-phosphat-dehydrogenase (GPD), etc. Bevorzugt ist der Cellobiohydrolase-I-, der Cellobiohydrolase-II-, der Amylase-, der Glucoamylase-, der Xylanase- oder der Enolase-Promotor.

Zwei Hauptmethoden zur Kontrolle der Expression sind bekannt, d.h. Überexpression und Unterexpression. Überexpression kann durch Insertion einer oder mehrerer Extra-Kopien des ausgewählten Gens erzielt werden. Für die Unterexpression gibt es zwei Hauptmethoden, die üblicherweise auf dem Fachgebiet als "Antisense Downregulation" und "Sense Downregulation" bezeichnet werden. Im Allgemeinen werden diese Verfahren als "Gene Silencing" bezeichnet. Beide dieser Methoden führen zu einer Hemmung der Expression des Targetgens.

Zusätzlich zur Verwendung eines speziellen Promotors können andere Typen von Elementen die Expression von Transgenen beeinflussen. Insbesondere wurde gezeigt, dass Introns das Potenzial zur Verstärkung der Transgenexpression besitzen.

Die Expressionskassette kann noch weitere Elemente umfassen, beispielsweise solche, die durch endogene oder exogene Elemente wie Zinkfinger-Proteine, einschließlich natürlich vorkommender Zinkfinger-Proteine oder chimerer Zinkfinger-Proteine, reguliert werden können.

Die erfindungsgemäß verwendete Expressionskassette kann ferner Enhancer-Elemente oder stromaufwärtige Promotor-Elemente enthalten.

Vektoren zur erfindungsgemäßen Verwendung können so konstruiert werden, dass sie ein Enhancer-Element enthalten. Die erfindungsgemäßen Konstrukte umfassen somit das Gen von Interesse zusammen mit einer 3'-DNA-Sequenz, die als Signal wirkt, um die Transkription zu terminieren und die Polyadenylierung der so erhaltenen mRNA zu erlauben. Es können beliebige Signalsequenzen verwendet werden die die Sekretion aus dem gewählten Wirtsorganismus ermöglichen. Bevorzugte Signalsequenz ist die Phytase-Signalsequenz aus *Aspergillus niger* oder daraus abgeleitete Signalsequenzen für die Sekretion aus filamentösen Pilzen.

Es kann auch eine spezielle Leadersequenz verwendet werden, da die DNA-Sequenz zwischen der Transkriptionsstartstelle und dem Start der codierenden Sequenz, d.h. der nicht-translatierten Leadersequenz die Genexpression beeinflussen kann. Bevorzugte Leadersequenzen umfassen Sequenzen, die die optimale Expression des angehefteten Gens steuern, d.h sie umfassen eine bevorzugte Consensus-Leader-Sequenz, die die mRNA-Stabilität erhöht oder erhält und eine ungeeignete Translationsinitiation verhindert. Die Wahl solcher Sequenzen ist einem Fachmann auf dem Gebiet gut bekannt.

Um die Möglichkeit, die Transformanten zu identifizieren zu verbessern, kann ein selektierbares oder screenbares Markergen in die Expressionskassette aufgenommen werden. Derartige Markergene sind einem Fachmann auf dem Gebiet gut bekannt.

Die Expressionskassette oder ein Vektorkonstrukt, das die Expressionskassette enthält, wird in eine Wirtszelle eingeführt. Eine Vielzahl von Techniken sind verfügbar und einem Fachmann auf dem Gebiet zur Einschleusung von Konstrukten in eine Wirtszelle gut bekannt. Die Transformation mikrobieller Zellen kann unter Verwendung von Polyethylenglykol, Calciumchlorid, viraler Infektion, DEAE-Dextran, Phageninfektionen, Elektroporation und anderen auf dem Fachgebiet bekannten Methoden durchgeführt werden. Die Transformation von Pilzen kann nach Penttilä et al., Gene 61:155-164, 1987 durchgeführt werden. Die Einschleusung eines rekombinanten Vektors in Hefen kann nach an sich bekannten Verfahren einschließlich Elektroporation, Verwendung von Spheroplasten, Lithiumacetat und dergleichen durchgeführt werden.

Sobald die erfindungsgemäße Expressionskassette bzw. DNA-Sequenz erhalten ist, kann sie in Vektoren nach an sich bekannten Verfahren eingefügt werden, um das codierte Polypeptid in geeigneten Wirtssystemen zu überexprimieren. Jedoch können auch DNA-Sequenzen als solche verwendet werden, um geeignete Wirtssysteme der Erfindung zu transformieren, um eine Überexpression des codierten Polypeptids zu erzielen.

Sobald eine erfindungsgemäße DNA-Sequenz in einer geeigneten Wirtszelle in einem geeigneten Medium exprimiert wurde, kann die codierte Phytase entweder aus dem Medium, falls die Phytase in das Medium sezerniert wird oder aus dem Wirtsorganismus, falls die Phytase intrazellulär z. B. im periplasmatischen Raum vorhanden ist, nach an sich bekannten Verfahren aufkonzentriert und/oder isoliert werden. Bekannte Verfahren der Abtrennung der unlöslichen Bestandteile des Kulturmediums und der Biomasse gefolgt von Verfahren zur Aufkonzentrierung der Phytase können zur Herstellung von konzentrierten Phytaselösungen oder als Vorbereitung zur Trocknung der Phytase angewendet werden. Beispielsweise können Filtrationsverfahren oder Zentrifugationsverfahren zur Abtrennung der unlöslichen Bestandteile verwendet werden gefolgt von Ultrafiltrationsverfahren zur Aufkonzentration oder es werden Cross-flow Filtrationsverfahren angewendet. Die Trocknung kann durch Gefrier- und Sprühtrocknen, Granulationsverfahren, Extrudierung oder andere Verfahren erfolgen. Bekannte Verfahren der Proteinreinigung können verwendet werden, um die erfindungsgemäßen Phytasen zu isolieren. Beispielsweise können verschiedene chromatographische oder gelchromatographische Verfahren einzeln oder in Kombination verwendet werden. In Abhängigkeit von der verwendeten Wirtszelle bei einem rekombinanten Produktionsverfahren kann das erfindungsgemäße Enzym kovalent durch Glykosilierung modifiziert sein oder nicht. In eukaryotischen Zellen dient die Glykosilierung der sezernierten Proteine dazu, die Proteinfaltung, die Konformationsstabilität, die thermische Stabilität und die Resistenz gegenüber Proteolyse zu modulieren. Im Hinblick auf eine spezifische Anwendung der Phytase kann eine glykosilierte Variante des Enzyms gegenüber einer nicht-glykosilierten Variante bevorzugt sein. Beispielsweise dient die Verwendung einer glykosilierten Phytase in Tierfutter zum Schutz des Enzyms vor thermischer Denaturierung während der Futterpelletisierung und vor proteolytischer Inaktivierung bei der Passage durch den Magen des Tieres, wodurch die Verteilung des aktiven Enzyms im Darmtrakt und an die Wirkstelle gefördert wird. Für Anwendungen in der Nahrungsmittelverarbeitung, bei der die Enzymaktivität nur während der Verarbeitung und nicht im Endprodukt erwünscht ist, kann eine thermolabile, d.h. nicht-glykosilierte, und gegenüber protolytischem Abbau empfindliche Phytase bevorzugt sein.

Die Erfindung betrifft auch Phytasezusammensetzungen, die das erfindungsgemäße Polypeptid enthalten. Im Allgemeinen sind Phytasezusammensetzungen flüssig oder trocken. Flüssige Zusammensetzungen enthalten das Phytaseenzym bevorzugt in einer gereinigten oder angereicherten Form. Jedoch können auch Hilfsstoffe wie beispielsweise ein Stabilisator mit Glycerin, Sorbit oder Monopropylenglykol, Additive wie Salze, Zucker, Konservierungsstoffe, Mittel zur Einstellung des pH-Werts, Proteine und Phytat oder Salze von Myoinositolphosphaten (ein Phytasesubstrat) zugesetzt werden. Typische Flüssigzusammensetzungen sind wässrige oder ölige Aufschlämmungen. Die Flüssigzusammensetzungen können einem Nahrungsmittel oder Futtermittel vor oder nach einer möglichen Pelletierung bzw. Verarbeitungsschritt zugesetzt werden. Trockenzusammensetzungen können gefriergetrocknete, sprühgetrocknete oder extrudierte Zusammensetzungen sein, die ausschließlich das Enzym enthalten können. Trockenzusammensetzungen können Granulate sein, die leicht beispielsweise mit Nahrungsmitteln oder Futterkomponenten gemischt werden können oder bevorzugter eine Komponente eines Prämix bilden. Die Teilchengröße des Enzymgranulats ist bevorzugt mit der der anderen Komponenten des Gemisches kompatibel. Dies ermöglicht sichere und zweckdienliche Mittel, um Enzyme beispielsweise in prozessierte Nahrungsmittel, Prämixe oder Tierfutter einzuarbeiten.

Beispielsweise kann eine stabile Formulierung des erfindungsgemäßen Phytaseenzyms dadurch hergestellt werden, dass ein Gemisch aus einer flüssigen Enzymlösung auf ein Füllmittel wie gemahlenes Sojabohnenmehl gesprüht wird und dann das Gemisch getrocknet wird. Die Verringerung der Feuchtigkeit und die Bindungswechselwirkungen der Phytase mit dem Füllmittel schützen das Enzym vor Umwelteinflüssen wie Temperaturextrema, die während der Herstellung des Futtermittels auftreten können. Trockene und flüssige Formulierungen können weiter stabilisiert werden, wenn die Aktivität potenzieller proteolytischer Enzyme verringert wird, die als Nebenprodukte in dem Flüssigfermentationsgemisch vorhanden sein können, das zur Herstellung des erfindungsgemäßen Enzyms verwendet wird. Das so erhaltene Trockenenzym-Gemisch kann als Futterergänzungsmittel zur Verwendung in der Geflügel- und Schweinezucht verwendet werden. Beispielsweise zeigt die Zugabe von 250 Enzymeinheiten des erfindungsgemäßen Enzyms zu 1 kg Standardweizendiät ähnliche Wirkungen wie 500 Enzymeinheiten *Aspergillus* Phytase. Ferner führt eine Verringerung der Phosphat-Supplementation zu einer Abnahme der PhosphatVerunreinigung, die ihrerseits signifikant die Umweltbelastung durch intensive Tierzucht verringert.

Sobald ein Trockenenzym-Präparat erhalten ist, kann ein Agglomerations-Granulat hergestellt werden. Hierfür wird ein Mischer mit hohen Scherkräften verwendet, wodurch Füllmaterial und das Enzym co-agglomerieren und ein Granulat gebildet wird. Absorptionsgranulate werden hergestellt, indem Kerne aus einem Trägermaterial durch das erfindungsgemäße Enzym beschichtet werden. Typische Füllmaterialien sind Salze wie Dinatriumsulfat. Andere Füllmaterialien umfassen Kaolin, Talk, Magnesiumaluminiumsilicat und Cellulosefasern. Gegebenenfalls werden Bindemittel wie Dextrine auch in das Agglomerationsgranulat aufgenommen.

Typische Trägermaterialien umfassen Stärke, z. B. in Form von Cassava, Kartoffel und Getreide, insbesondere Mais, Reis und Weizen, oder proteinhaltige Erzeugnisse wie z.B. Sojaprotein. Salze können auch verwendet werden. Das Granulat wird gegebenenfalls mit einem Beschichtungsgemisch umhüllt. Ein solches Gemisch umfasst Beschichtungsmittel, bevorzugt hydrophobe Beschichtungsmittel, dehydriertes Palmöl und Talk und gegebenenfalls andere Additive wie Calciumcarbonat oder Kaolin um die Bioverfügbarkeit an der bestimmungsgemäßen Wirkstätte zu verbessern.

Zusätzlich können die Mischungen mit Phytase andere Stoffe wie farbgebende Mittel, Aromaverbindungen, Stabilisatoren, Vitamine, Mineralien, andere Nahrungsmittel- oder Futter-Enzyme und dergleichen enthalten. Dies trifft insbesondere für die sogenannten Prämixe zu.

Ein Nahrungsmittel- oder Futtermitteladditiv ist eine im Wesentlichen reine Verbindung oder eine Zusammensetzung aus mehreren Verbindungen, die dazu bestimmt oder geeignet ist Nahrungsmitteln oder Futtermitteln zugesetzt zu werden. Insbesondere ist es eine Substanz, die gemäß ihrem Verwendungszweck eine Komponente eines Nahrungsmittels oder Futtermittels wird oder Eigenschaften eines Nahrungsmittel- oder Futtermittelprodukts beeinflusst. So soll ein Phytaseadditiv eine Phytase bezeichnen, die kein natürlicher Bestandteil der hauptsächlich für Nahrungsmittel und Futtermittel verwendeten Substanzen ist oder in ihrer natürlichen Konzentration darin nicht vorhanden ist. Beispielsweise wird die Phytase dem Futtermittel separat von den Futtermittelsubstanzen allein oder in Kombination mit anderen Futtermitteladditiven zugesetzt. Ein typischer Prämix umfasst üblicherweise eine oder mehrere Verbindungen wie Vitamine, Mineralien oder Futter-verstärkende Enzyme und geeignete Träger und/oder Exzipienzien.

Ein gebrauchsfertiges Phytaseadditiv ist ein Additiv, das nicht in situ in Tierfutter oder in verarbeiteten Nahrungsmitteln produziert wird. Ein gebrauchsfertiges Phytaseadditiv kann Menschen oder Tieren direkt oder bevorzugt direkt nach dem Mischen mit anderen Bestandteilen des Futters oder der Nahrungsmittel verabreicht werden. Beispielsweise wird ein Futteradditiv gemäß diesem Aspekt der vorliegenden Erfindung mit anderen Futtermitteln und -additiven unter Erhalt eines Prämixes oder Ergänzungsfutters vereinigt. Solche anderen Futtermittelbestandteile umfassen eine oder mehrere andere (bevorzugt thermostabile) Enzymsupplemente, andere Futteradditive, mineralische Futtermittel und Aminosäuren. Die so erhaltenen (kombinierten) Futteradditive können mehrere verschiedene Verbindungstypen umfassen und können dann in ihrer geeigneten Menge mit den Futtermitteln wie Getreide- und Proteinträgern unter Bildung eines zusammengesetzten Tierfutters vermischt werden. Die Verarbeitung dieser Komponenten zu einem Tierfutter kann nach dem Mischen mit an sich bekannten Verarbeitungsvorrichtungen wie einer Doppelpelletiermaschine, eines Dampfpelletierers, eines Expanders oder eines Extruders durchgeführt werden.

Auf ähnliche Weise kann ein Nahrungsmitteladditiv gemäß dieser Ausführungsform der vorliegenden Erfindung mit anderen Nahrungsmittelkomponenten vereinigt werden, wodurch verarbeitete Nahrungsmittelprodukte erzeugt werden. Solche anderen Nahrungsmittelkomponenten umfassen ein oder mehrere Enzymsupplemente, Vitamine, Mineralien und Spurenelemente. Das so erhaltene kombinierte Nahrungsergänzungsmittel kann dann in einer geeigneten Menge mit anderen Nahrungsmittel-Komponenten wie Getreide und Pflanzenproteine vermischt werden, um ein verarbeitetes Nahrungsmittel zu ergeben. Die Verarbeitung dieser Komponenten zu einem verarbeiteten Nahrungsmittel kann unter Verwendung an sich bekannter Verarbeitungsvorrichtungen durchgeführt werden.

In einer bevorzugten Ausführungsform umfassen die erfindungsgemäßen Phytasezusammensetzungen zusätzlich eine wirksame Menge eines oder mehrerer Enzyme für Nahrungs- oder Futtermittel, bevorzugt ausgewählt aus alpha-Galactosidasen, beta-Galactosidasen, Laccasen, anderen Phytasen, Phosphatasen, Endoglucanasen, insbesondere endo-beta-1,4-Glucanasen, endo-beta-1,3(4)-Glucanasen, endo-1,2-beta-Glucanasen und endo-1,3-alpha-Glucanasen, Cellulasen, Xylosidasen, Galactanasen, insbesondere Arabinogalactan-endo-1,4-beta-Galactosidasen und Arabinogalactan-endo-1,3-beta-Galactosidasen, Pectin-abbauenden Enzymen, insbesondere Pectinasen, Pectinesterasen, Pectinlyasen, Polygalacturonasen, Arabananasen, Rhamnogalacturonasen, Rhamnogalacturonanacetylesterasen, Rhamnogalacturonan-alpha-Rhamnosidasen, Pectatlyasen und alpha-Galacturonidasen, Mannanasen, beta-Mannosidasen, Mannanacetylesterasen, Xylanacetylesterasen, Proteasen, Xylanasen, Arabinoxylanasen und lipolytischen Enzymen wie Lipasen, Phospholipasen und Cutinasen.

Das erfindungsgemäße Tierfutteradditiv wird dem Tier vor oder gleichzeitig mit dem Futter verabreicht. Bevorzugt wird das erfindungsgemäße Tierfutteradditiv dem Tier gleichzeitig mit dem Futter verabreicht.

Eine effektive Menge an Phytase in Nahrungsmitteln oder Futtermitteln beträgt etwa 10 - 20.000 PPU/kg, bevorzugt etwa 10 - 15.000 PPU/kg, bevorzugter etwa 10 - 10.000 PPU/kg, insbesondere etwa 50 - 5.000 PPU/kg, insbesondere 50 - 2.000 PPU/kg Futter oder Nahrungsmittel.

Die Erfindung betrifft auch die Verwendung von Phytase zur Verarbeitung und Herstellung von Nahrungsmitteln und Futtermitteln. Getreide und Mehle für Nahrungsmittel können enzymatisch mit Phytase behandelt werden, um den Phytingehalt der Rohstoffe zu verringern. Verringerte Phytingehalte verbessern die Nahrungsmittelqualität, indem die Verfügbarkeit essenzieller Mineralien wie Eisen, Calcium und Zink erhöht wird. Zusätzlich zur Erhöhung der Qualität der Nahrungsmittel kann die Verwendung von Phytase während der Verarbeitung die Gesamteffizienz der Nahrungsmittelherstellung verbessern. Beispielsweise kann die Zugabe von Phytase zu weißen Sojabohnenflocken während der Herstellung eines Sojaproteinisolats signifikant die Ausbeute und Qualität des extrahierbaren Proteins erhöhen. Dabei ist die Phytase nur während der Herstellung und Verarbeitung aktiv und ist in dem Endprodukt nicht mehr aktiv. Dieser Aspekt ist insbesondere bei der Teigherstellung und beim Backen und der Produktion anderer vetzehrfertiger Produkte auf Getreidebasis von Bedeutung. Auf ähnliche Weise können Tierfutterkomponenten wie getoastetes Sojabohnenmehl oder Canolamehl mit Phytase vor dem eigentlichen Herstellungsprozess vorbehandelt werden. Die Entfernung von anti-nutritiven Faktoren in Tierfutterkomponenten vor der Herstellung führt zu einer physiologisch höheren Qualität und zu wertvolleren Tierfutterinhaltsstoffen. Bei diesen Verarbeitungsverfahren ist die Phytase während der Herstellung aktiv und ist im Verdauungstrakt des Tiers nach Aufnahme des behandelten Futters in der Regel nicht mehr aktiv.

Zusätzlich zur Verwendung von Phytase als Futterverarbeitungshilfsmittel betrifft die vorliegende Erfindung die Verwendung der erfindungsgemäßen Phytase als Verdauungshilfe. Phytase in Tablettenform kann zusammen mit der Nahrungsaufnahme eingenommen werden, um das aktive Enzym im Magen-Darm-Trakt zu verteilen.

Die erfindungsgemäße Phytase kann ebenso in vorteilhafter Weise bei monogastrischen wie polygastrischen Tieren, insbesondere bei jungen Kälbern zum Einsatz kommen. Futter für Fische und Schalentiere kann ebenfalls mit Phytase supplementiert werden, um die Ausnutzung des Futters zu verbessern und den Gehalt an ausgeschiedenem Phosphor bei intensiver Tierzucht zu verringern. Das erfindungsgemäße Futtermittel kann auch Tieren wie Geflügel, z. B. Masthühnern, Truthühnern, Gänsen, Enten sowie Schweinen, Pferden, Rindern, Schafen, Ziegen, Hunden und Katzen sowie Fischen und Schalentieren verabreicht werden. Es ist jedoch besonders bevorzugt, dass das erfindungsgemäße Futtermittel Schweinen oder Geflügel verabreicht wird.

Erfindungsgemäße Phytaseformulierungen können auch mit anderen Inhaltsstoffen kombiniert werden, wodurch neue und besonders vorteilhafte Futterzusammensetzungen entstehen. Da, wie vorstehend ausgeführt, die Verfügbarkeit von pflanzlichem Phosphat, das in Sojabohnenmehl und Getreide in Folge der Bindung an Phytinsäure niedrig ist, wird anorganisches Phosphat dem Futter zugesetzt, um eine adäquate Phosphorversorgung der Tiere zu gewährleisten. Jedoch enthalten diese Futtermittel zu viel Gesamtphosphat und führen dadurch zu einer Verschmutzung der Umwelt mit Phosphat. Speziell umfasst das erfindungsgemäße Tierfuttermittel die Kombination einer erfindungsgemäßen Phytase mit Tierfutterinhaltsstoffen, um ein Futtermittel zu ergeben, das wesentlich erniedrigte Gehalte an zugesetztem anorganischem Phosphor enthält. In einer bevorzugten Ausführungsform umfasst das erfindungsgemäße Futtermittel typische Futterinhaltsstoffe, Mikronährstoffe, Spurenelemente, Vitamine, etc. sowie eine effektive Menge an Phytase und anorganischem Phosphor, wobei die Mengen der Phytase und des Phosphors zwischen 50 und 20.000 Einheiten Phytase/kg Futtermittel und weniger als 0,45% anorganischem Phosphor, bevorzugt zwischen Gehalten von 100 - 10.000 Einheiten Phytase/kg Futtermittel und weniger als 0,225% anorganischem Phosphor, insbesondere Gehalte von 150 - 10.000 Einheiten Phytase/kg Futtermittel und weniger als 0,15% anorganischem Phosphor, insbesondere Gehalte von 200 - 20.000 Einheiten Phytase/kg Futtermittel und keinen zusätzlichen Zusatz an anorganischem Phosphor betragen.

Die Erfindung betrifft auch Verfahren zur Verbesserung der Gewichtszunahme und des Futterverwertungsvermögens (Feed Conversion Ratio, FCR) in der Tierernährung sowie die Verwendung der erfindungsgemäßen Phytasen in diesen Verfahren. Eine erfindungsgemäße Phytase erlaubt verbesserte Gewichtszunahmen und ein verbessertes Futterverwertungsvermögen, insbesondere im Zusammenhang mit Futter, das wenig anorganisches Phosphat enthält. Gemäß den erfindungsgemäßen Verfahren kann der Gehalt an anorganischem Phosphat in Futter unter Gehalte von 0,45%, bevorzugt unter 0,225%, gesenkt werden. Bevorzugt wird kein anorganisches Phosphat zugesetzt. Durch eine erhöhte Phosphat-Verfügbarkeit in Folge des Zusatzes des erfindungsgemäßen Enzyms kann die Knochenmineralisation der Tiere signifikant verbessert werden, was insbesondere bei rasch wachsenden Tieren von großer Bedeutung ist.

Gemäß einer noch weiteren Ausführungsform betrifft die Erfindung die Verwendung des erfindungsgemäßen Enzyms beim Backen, wodurch Entwicklung, Elastizität und/oder Stabilität des Teigs und/oder das Volumen, die Krumenstruktur und/oder der Widerstand des Backguts gegenüber dem Altbackenwerden verbessert wird. Obwohl das erfindungsgemäße Enzympräparat zur Herstellung von Teig oder gebackenen Produkten aus beliebigen Typen von Mehl, z. B. auf der Basis von Roggen, Gerste, Hafer oder Mais verwendet werden kann, erwies sich das erfindungsgemäße Enzympräparat als besonders nützlich bei der Herstellung von Teigen oder Backprodukten aus Weizen oder aus einem wesentlichen Weizenanteil. Die Backprodukte, die mit einem erfindungsgemäßen Enzympräparat hergestellt werden können, umfassen Brot, Brötchen, Baguette und dergleichen. Zum Backen kann das erfindungsgemäße Enzympräparat mit einer weiteren Enzymaktivität wie z. B. Xylanase, Lipase, Amylase, Oxidase oder Laccase neben der Phytase verwendet werden oder kann in Kombinationen mit weiteren Enzymen wie Lipase, Amylase, Oxidase (z. B. Glucoseoxidase, Peroxidase), verwendet werden.

Die nachstehenden Beispiele erläutern die Erfindung näher.

### Beispiel 1: Bestimmung der Phytaseaktivität

Die Phytaseaktivität wurde in einem Assay-Gemisch aus 0,5% Phytinsäure (etwa 5 mM), 200 mM Natriumcitrat, pH 5,0, gemessen. Nach 15-minütiger Inkubation bei 37°C wurde die Reaktion durch Zugabe eines gleichen Volumens 15% Trichloressigsäure gestoppt. Die freigesetzten Phosphationen wurden durch Mischen von 100 µl des Assay-Gemisches mit 900 µl H₂O und 1 ml 0,6 M H₂SO₄, 2% Ascorbinsäure und 0,5% Ammoniummolybdat nach Inkubation bei 50°C und einer Dauer von 20 min, bei 820 nm quantitativ bestimmt. Kaliumphosphat-Standardlösungen wurden als Referenz verwendet.

### Beispiel 2: Konstruktion der Plasmide pKDa2 und pKDa4

Die Phytase codierende Sequenz von *E*. *coli* (Dassa et al. 1990, J. Bacteriol. 172:5497-5500, Accessions-Nr. M58704) wurde entwickelt und unter Verwendung des Codon-Gebrauchs von *T. reesei* (http://www.kazusa.or.op/codon) synthetisiert. Alle synthetisierten Fragmente wurden sequenziert und Fragmente mit und ohne Mutationen wurden assoziiert, wodurch neue Phytasevarianten produziert wurden. In einer der abschließend erhaltenen Varianten wurde die Aminosäure Val²⁰⁰ (GTG) des Phytasegens zu Tyr²⁰⁰ (TAC) geändert. Diese Variante wurde als Da2 bezeichnet. Das nicht mutierte Stammpolynucleotid wurde als Da4 bezeichnet. Die reifen *E. coli*-Phytase-Genklone wurde mittels PCR amplifiziert. Die DNA-Sequenz mit dem CAG (Gln)-Codon in Position 1 umfasst ein offenes Leseraster von 1230 bp und codiert ein Enzym mit 410 Aminosäuren (SEQ ID NO: 1/2).

Das Signalpeptid (18 Aminosäuren) der *A*. *niger* Phytase (SEQ ID NO: 3/4) wurde verwendet, um die *E. coli*-Phytase aus *Trichoderma reesei* zu sezernieren. Das synthetische Gen mit der modifizierten *A. niger* Phytase Signalsequenz und der reifen *E. coli*-Phytasesequenz die die Mutation V200Y enthält wurde in das Plasmid pUC18 cloniert. Der entstandene Vektor wurde Da2pUC3 genannt und als DSM 16396 gemäß der vorstehenden Bedingungen hinterlegt. Das hinterlegte Plasmid enthält die DNA mit der Pilz Condon usage mit geringfügigen Modifikationen entsprechend den benötigten Schnittstellen für die Restriktionsenzyme wie aus folgender Tabelle 1 hervorgeht:

**Tabelle 1:**

| **T. reesei** | | | **Synthetisches Phytase Gen** | |
|---|---|---|---|---|
| Pilz usage | Codon | Frequenz pro Tausend | Anzahl triplets in synthetischem *E*. *coli* Phytase Gen | Frequenz pro Tausend in synthetischem *E. coli* Phytase Gen |
| **Ala** | **GCC** | **43,3** | **20** | **48,8** |
| Ala | GCT | 19,1 | 10 | 24,4 |
| Ala | GCG | 16,0 | 4 | 9,8 |
| Ala | GCA | 10,3 | 3 | 7,3 |
| **Arg** | **CGC** | **14,3** | **12** | **29,3** |
| Arg | CGT | 7,0 | 6 | 14,6 |
| Arg | CGA | 6,4 | 0 | 0,0 |
| Arg | AGG | 5,5 | 1 | 2,4 |
| Arg | CGG | 5,1 | 3 | 7,3 |
| Arg | AGA | 2,5 | 0 | 0,0 |
| **Asn** | **AAC** | **43,3** | **13** | **31,7** |
| Asn | AAT | 10,3 | 3 | 7,3 |
| **Asp** | **GAC** | **37,5** | **14** | **34,1** |
| Asp | GAT | 15,7 | 5 | 12,2 |
| **Cys** | **TGC** | **12,8** | **5** | **12,2** |
| Cys | TGT | 4,0 | 3 | 7,3 |
| **Gln** | **CAG** | **37,1** | **24** | **58,5** |
| Gin | CAA | 8,6 | 5 | 12,2 |
| **Glu** | **GAG** | **31,1** | **17** | **41,5** |
| Glu | GAA | 6,9 | 4 | 9,8 |
| **Gly** | **GGC** | **54,4** | **16** | **39,0** |
| Gly | GGT | 16,9 | 6 | 14,6 |
| Gly | GGA | 13,0 | 6 | 14,6 |
| Gly | GGG | 8,2 | 1 | 2,4 |
| **His** | **CAC** | **18,3** | **7** | **17,1** |
| His | CAT | 4,0 | 1 | 2,4 |
| **Ile** | **ATC** | **29,9** | **11** | **26,8** |
| Ile | ATT | 15,1 | 3 | 7,3 |
| Ile | ATA | 1,5 | 0 | 0,0 |
| **Leu** | **CTG** | **26,1** | **27** | **65,9** |
| Leu | CTC | 25,1 | 21 | 51,2 |
| Leu | CTT | 9,9 | 5 | 12,2 |
| Leu | TTG | 6,7 | 0 | 0,0 |
| Leu | CTA | 1,8 | 0 | 0,0 |
| Leu | TTA | 0,3 | 0 | 0,0 |
| **Lys** | **AAG** | **38,5** | **13** | **31,7** |
| Lys | AAA | 3,4 | 1 | 2,4 |
| **Met** | **ATG** | **18,8** | **5** | **12,2** |
| **Phe** | **TTC** | **21,3** | **7** | **17,1** |
| Phe | TTT | 13,5 | 4 | 9,8 |
| **Pro** | **CCC** | **23,3** | **14** | **34,1** |
| Pro | CCT | 15,0 | 9 | 22,0 |
| Pro | CCG | 13,4 | 6 | 14,6 |
| Pro | CCA | 7,1 | 0 | 0,0 |
| **Ser** | **TCC** | **21,6** | **8** | **19,5** |
| Ser | AGC | 21,3 | 9 | 22,0 |
| Ser | TCG | 19,3 | 5 | 12,2 |
| Ser | TCT | 14,0 | 4 | 9,8 |
| Ser | TCA | 6,4 | 0 | 0,0 |
| Ser | AGT | 4,1 | 0 | 0,0 |
| **Thr** | **ACC** | **29,0** | **18** | **43,9** |
| Thr | ACG | 20,6 | 10 | 24,4 |
| Thr | ACT | 14,0 | 5 | 12,2 |
| Thr | ACA | 6,3 | 0 | 0,0 |
| **Trp** | **TGG** | **17,6** | **8** | **19,5** |
| **Tyr** | **TAC** | 27,1 | 4 | 9,8 |
| Tyr | TAT | 9,0 | 1 | 2,4 |
| **Val** | **GTC** | **36,3** | **14** | **34,1** |
| Val | GTG | 14,8 | 5 | 12,2 |
| Val | GTT | 11,7 | 4 | 9,8 |
| Val | GTA | 2,2 | 0 | 0,0 |

In dem Plasmid pKDa2 (Tyr²⁰⁰-Mutante) ist das synthetische Gen durch eine SacII Restriktionsspaltstelle 16 Basenpaare stromaufwärts des Startcodons und eine *Bam*HI Restriktionsspaltstelle unmittelbar stromabwärts des Stopcodons flankiert. Die 16 Basenpaare stromaufwärts des Startcodons gehören zu dem *T. reesei cbh*I Promotor (Shoemaker et al., 1983, Bio/Technology 1, 691-696). Das synthetische Gen wurde mit *Sac*II und *Bam*HI gespalten und in die *Sac*II und *Bam*HI Spaltstellen nach dem *T. reesei Cellobiohydrolase* I Promotor in dem Plasmid pALK487 (WO 94/28117) inseriert. Dieses Plasmidkonstrukt wurde als pKDa1 bezeichnet. Ein glattendiges 4,78 kb langes *EcoR*I/*Spe*I-Fragment des Plasmids pALK424 (WO 93/24621), das den *amdS* Marker und die 3'-flankierenden *cbh*I-Sequenzen enthielt, wurde in die *Stu*I Spaltstelle von pKDa1 cloniert, wodurch der Phytaseexpressionsvektor pKDa2 erhalten wurde. Der Vektor wurde durch Restriktionsendonucleasen kartiert und die vollständige Sequenz des synthetisierten Fragments wurde durch Sequenzieren bestätigt.

In analoger Weise wurde die Konstruktion des Expressionsvektors pKDa4 (Wildtyp) durchgeführt.

Die aus den Plasmiden pKDa2 bzw. pKDa4 isolierten Expressionskassetten enthalten das folgende genetische Material:
*Cbh*I (Cellobiohydrolase I) Promotor: Das 2,2 kb *EcoR*I/*Sac*II-Fragment, das den cbhI-Promotor enthält, stammt von *Trichoderma reesei* QM6a. Der Promotor-Bereich funktioniert auch als homologe DNA (zusammen mit dem *cbh*I *3'-*Fragment; siehe nachstehend), um die Integration der transformierenden DNA in den *cbh*I Lokus zu steuern.

Signalsequenz: Das Signalpeptid der *A*. *niger Phytase* (SEQ. ID. NO.: 3/4) wurde verwendet, um die *E. coli*-Phytase aus *Trichoderma reesei* zu sezemieren.

*E. coli*-Phytase-Gen: Synthetisches *E. coli*-Phytase-Gen (SEQ ID NO: 1) inkl. der modifizierten *A*. *niger* Phytase Signalsequenz zur Expression in *T*. *reesei* wurde zwischen den *cbh*I Promotor und den *cbh*I Terminator fusioniert.

*cbh*I Terminator: Das 0,75 kb lange *Bam*HI/*Stu*I-Fragment, das den *cbh*I Terminator enthält, wurde im Anschluss an die *E. coli*-Phytase hinzugefügt, um die Termination der Transkription zu gewährleisten.

*amdS*-Gen: Das Gen inkl. seines Promotors und seines Terminators wurde aus *Aspergillus nidulans* VH1-TRSX6 isoliert und codiert für Acetamidase (Hynes et al., 1983, Mol. Cell. Biol. 3: 1430-1439; Kelly and Hynes, 1985, EMBO J. 4:475-47). Acetamidase erlaubt dem Stamm unter Verwendung von Acetamid als alleiniger Stickstoffquelle zu wachsen und dieses Charakteristikum wurde zur Selektion der Transformanten verwendet. Das 3,1 kb große Fragment (glattendig *Spe*I/*Bam*HI) enthält 1007 bps des Promotorbereichs, 1897 bps der codierende Region (einschließlich Introns) und 183 bps Terminatorbereich des *amdS*-Gens.

*cbh*I 3' Fragment: Das Fragment (1,7 kb, *Bam*HI/*Eco*RI, beginnend 1,4 kb nach dem Stopcodon des Gens) wurde aus *T*. *reesei* ALKO2466 isoliert. Der Stamm ALKO2466 stammt von dem Stamm ALKO233 (Harkki et al., 1991, Enzyme Microb. Technol. 13: 227-233) ab. Das 3'-Fragment wird zusammen mit dem Promotorbereich verwendet, um die Phytase-Expressionskassette in den *cbh*I Lokus durch homologe Rekombination zielgerecht zu integrieren.

Das Konstrukt wurde ausgewählt, um das Einzelkopie-Gen *cbh*I, das in *T*. *reesei* RH3780d vorhanden ist, zielgerecht aufzufinden und zu ersetzen, um die Wirkung der Mutation mittels einer Einzelkopie des Gens zu studieren.

### Beispiel 3: Transformation von Trichoderma reesei mit pKDa2 und pKDa4, um Einzelkopie-Transformanten zu erhalten.

*T. reesei* RH 3780d wurde mit den aus den Plasmiden pKDa2 und pKDa4 isolierten linearisierten Expressionskassetten separat transformiert. Die zur Transformation und zur Handhabung von *T*. *reesei* verwendeten Techniken waren die gemäß Penttilä et al. (1987, Gene 61: 155-164). Die Transformanten wurden selektiert und zweimal durch Einzelsporenisolierung gereinigt. Von allen Transformanten wurde diejenigen mit der höchsten Sekretionsleistung ausgewählt. Von diesen wurden Transformanten mit DNA aus dem Plasmid pKDa2 als RH 31068 und RH 31069 bezeichnet, Transformanten mit DNA aus pKDa4 wurden als RH 31071-31075 bezeichnet und für die weitere Charakterisierung herangezogen.

### Beispiel 4: Sekretion von Phytase in Schüttelkolben

Transformanten, die die Expressionskassetten von pKDa2 bzw. pKDa4 tragen, wurden im Schüttelkolben auf Cellulase-induzierendem Medium folgender Zusammensetzung gezüchtet: Milchprotein-Konzentrat Nutrica 2%, Lactose 1%, DSG 1,5%, KH₂PO₄ 5%, (NH₄)₂SO₄ 0,5%, Maisquellpulver 0,5%, Leitungswasser Rest, Einstellung des pH-Werts vor der Sterilisation auf 5,3. Die nach 6-tägiger Züchtung erhaltenen Kulturfiltrate wurden für die SDS-PAGE-Analyse und zur Bestimmung der Phytaseaktivität verwendet. Die Ergebnisse zeigten, dass die höchsten Phytaseaktivitäten im Kulturmedium mit Transformanten, die die pKDa2-Expressionskassette enthielten, beobachtet wurden. Die in der nachstehenden Tabelle 2 aufgeführten Aktivitäten sind die Maximal-Aktivitäten, die bei der Fermentation der besten Transformanten in dem Zeitraum von 6 Tagen erzielt wurden.

**Tabelle 2: Produktion von E. coli-Phytase durch Transformanten, die entweder die pKDa2- oder pKDa4-Expressionskassette enthalten.**

| **Stamm** | **SDS-PAGE** | **Southern-blot-Analyse Integrationsereignis** | **Phytase-Kassette Kopieanzahl** | **Phytase PPU g⁻¹** | **Expressionskassette** |
|---|---|---|---|---|---|
| RH31068 | CBHI⁻ | *cbh*I Lokus | eine Kopie | 417 | pKDa2 |
| RH31069 | CBHI⁻ | *cbh*I Lokus | eine Kopie | 411 | pKDa2 |
| | | | | | |
| RH31071 | CBHI⁻ | *cbh*I Lokus | eine Kopie | 188 | pKDa4 |
| RH31072 | CBHI⁻ | *cbh*I Lokus | eine Kopie | 182 | pKDa4 |
| RH31073 | CBHI⁻ | *cbh*I Lokus | eine Kopie | 171 | pKDa4 |
| RH31074 | CBHI⁻ | *cbh*I Lokus | eine Kopie | 167 | pKDa4 |
| RH31075 | CBHI⁻ | *cbh*I Lokus | eine Kopie | 158 | pKDa4 |
| | | | | | |
| RH3780d | CBHI⁺ | | | 0,7 | |

Die vorstehenden Ergebnisse zeigen, dass die mit der Expressionskassette pKDa2, die die Tyr²⁰⁰-Mutante enthält, transformierten Stämme eine etwa doppelt so hohe Phytasesekretion aufweisen wie mit der *E. coli*-Wildtyp-Sequenz, pKDa4, transformierten Stämme.

### Beispiel 5: Southern Blotting

Die Southern-Blot-Analyse wurde auf genomischen DNAs, die aus dem Wirtsstamm RH3780d und Transformanten beider Konstruktionen isoliert worden waren, durchgeführt, um das Integrationsereignis der Expressionskassette in das Genom zu bewerten. Nach Spaltung der genomischen DNA mit *Eco*RI und Durchmustern mit einem 9,0 kb *Eco*RI-Fragment war eine hybridisierende Bande zu 9,0 kb in allen Transformanten vorhanden. Die Größe dieser Bande entsprach dem 9,0 kb-Fragment der Expressionskassette, was eine intakte Integration der vollständigen Kassette in das Genom nahe legt.

Die Spaltung der DNA mit *Xba*I zeigte zwei hybridisierende Banden zu 1,7 und 9,0 kb in dem Wirtsstamm, wohingegen in allen Transformanten drei hybridisierende Banden zu 1,7, 4,0 und 7,0 kb vorhanden waren. Wie aus dem doppelten Crossover-Ereignis erwartet, führte eine Integration einer Kopie der Expressionskassette in den *cbh*I Lokus zu drei Banden zu 1,7, 4,0 und 7,0 kb und der Abwesenheit der 9,0 kb Wildtyp-Bande. Sowohl die 4,0 als auch die 7,0 Banden ersetzten die 9,0 Bande des *cbh*I Lokus des Wirts und die 1,7 kb Bande war unverändert.

Die Intensitäten der hybridisierten Signale zwischen den Transformanten und beiden Konstruktionen unterschieden sich nicht.

Die Ergebnisse der Southern-Blot-Analyse, SDS-PAGE und Bestimmung der Phytaseaktivitäten sind in der vorstehenden Tabelle 2 gezeigt. Die Southem-Blot-Analyse zeigte, dass alle selektierten Transformanten das *E. coli*-Phytase-Gen, bzw. das mutierte Gen, als Einzelkopie in dem *cbh*I Targetlokus enthielten.

### Beispiel 6: Biochemische Charakterisierung der Phytasevarianten

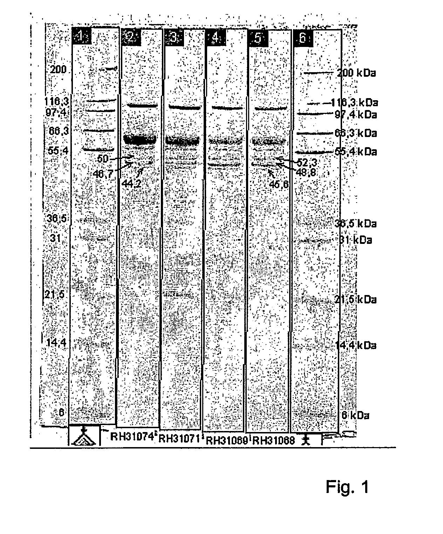

Überstände aus rekombinanten Stämmen wurden auf NuPage BisTris 10% SDS-PAGE-Gel getrennt und mit Coomassie gefärbt (Fig. 1). In Folge der Glykosilierung durch den Wirtsstamm erschien die Phytase als drei Banden zwischen 44,2 und 53,2 kDa. Alle Proben wurden mit gleichen Phytaseaktivitäten aufgetragen. Die Gele wurden getrocknet und mit der Phoretix ID Advanced Software auf einem Agfa Flachbettscanner gescannt. Die Flächen der drei Banden wurden integriert und die Daten sind in der nachstehenden Tabelle 3 zusammengefasst. Die Summe der Flächen aller drei Phytase-Banden ist für alle vier getesteten Stämme im Rahmen der Messgenauigkeit der Methode gleich. Dies zeigt, dass die Mutation in Da2 keine Veränderungen in der spezifischen Aktivität des Enzyms verursacht und belegt somit, dass die Mutation für die erhöhte Sekretion des Phytaseproteins verantwortlich ist.

**Tabelle 3: Integration der Fläche der Phytase-Banden auf dem SDS-PAGE-Gel unter Verwendung des Programms Phoretix ID Advanced**

| | | RH 31074 (pKDa 4) | RH 31071 (pKDa4) | RH 31069 (pKDa2) | RH 31068 (pKDa2) |
|---|---|---|---|---|---|
| Bande | MG | Bahn 2 | Bahn 3 | Bahn 4 | Bahn 5 |
| 1 | ca. 50, 52,3 kDa | 56676016 | 52581584 | 42442918 | 41508017 |
| 2 | ca. 46,7, 48,8 kDa | 35235004 | 34577257 | 47107624 | 43668804 |
| 3 | ca. 44,2, 45,6 kDa | 18121276 | 16714340 | 15716570 | 13439975 |
| | | | | | |
| Summe | | 110032296 | 103873181 | 105267112 | 98616796 |
| | | | | | |
| Gesamtprotein /IBahn [ug] | | 2,04 | 1,49 | 0,85 | 0,78 |

### Beispiel 7: Verbesserung der Verfügbarkeit von Phosphat durch die erfindungsgemäße Phytase (Tyr²⁰⁰-Mutante) bei Schweinen

Ein Verdauungsversuch wurde mit Schweinen in zwei aufeinander folgenden Sammelperioden zu 5 Tagen nach einer Anpassungsperiode von jeweils 9 Tagen durchgeführt. Eine Ration mit reduziertem Gehalt an verdaulichem Phosphat auf der Basis von Mais- und Sojaextraktionsschrot wurde als Negativkontrolle verwendet und mit der erfindungsgemäßen Phytase (*E*. *coli*-Phytasemutante Tyr²⁰⁰) in Mengen von 125, 250, 500 und 750 PPU/kg Futter supplementiert. Phytase wurde über eine Vormischung dem Gesamtfutter zugesetzt. Es wurden insgesamt 40 männliche kastrierte Schweine in 5 Behandlungsgruppen eingesetzt. Alle Behandlungsgruppen bestanden aus 8 Schweinen (4 Schweine in 2 aufeinanderfolgenden Sammelperioden). Die Behandlungen waren wie folgt:
Negativkontrolle¹ (NC)
   ¹Futter: 71,5% Maismehl, 28,8% Sojaextraktionsschrot
NC + Phytase (125 PPU kg⁻¹)
NC + Phytase (250 PPU kg⁻¹)
NC + Phytase (500 PPU kg⁻¹)
NC + Phytase (750 PPU kg⁻¹)
4,4 g kg⁻¹ gesamt P; 1,9 g kg⁻¹ Nicht-Phytat P; 5,5 g kg⁻¹ Ca; native Phytase: 90 PPU kg⁻¹

Die gemessenen Parameter umfassen Phosphat- und Calcium-Verdaulichkeit sowie Phosphat- und Calcium-Retention.

### Phosphat-Retention und -Exkretion:

Die Ergebnisse zeigen, dass bei allen Zugabemengen an Phytase (125; 250; 500 und 750 PPU kg⁻¹ Futter) die Phosphatverdaulichkeit signifikant von 42,5% in der Negativkontrolle auf 59,3, 65,3, 65,0 bzw. 66,3% (p<0,05) erhöht war. Dies führte zu einer signifikanten Abnahme der fäkalen Phosphatausscheidung von 203 mg kg⁻¹ 0,75 d⁻¹, gemessen in der Negativkontrollgruppe, auf 142, 120, 121 und 116 mg kg⁻¹ 0,75 d⁻¹ bei den Behandlungsgruppen mit Phytase angereichertem Futter (p<0,05). Die Phosphat-Retention verbesserte sich signifikant von 152 mg kg⁻¹ 0,75 d⁻¹, gemessen in der Negativkontrolle, auf 212, 233, 231 und 236 mg kg⁻¹ 0,75 d⁻¹ bei den Behandlungsgruppen mit Phytase angereichertem Futter (p<0,05). Die Unterschiede zwischen den Phytasebehandlungen waren signifikant zwischen der niedrigsten Dosierung (125 PPU kg⁻¹ Futter) und allen anderen Verabreichungen (p<0,05).

### Calcium-Retention und -Exkretion

Der Zusatz von erfindungsgemäßer Phytase erhöhte verglichen mit der Kontrollgruppe signifikant (p<0,05) die Calcium-Verwertung um 12,1, 14,3, 15,2 und 14,6%. Die Calciumausscheidung über den Urin war relativ hoch (114 mg kg⁻¹ 0,75 d⁻¹) in der Negativkontrolle, was vermutlich auf den niedrigen P-Gehalt in dem Futter zurückzuführen war. In den Phytase-Behandlungsgruppen wurde die Calcium-Ausscheidung über den Urin auf 108, 118, 95 und 88 mg kg⁻¹ 0,75 d⁻¹ verringert, was verglichen mit der Negativkontrolle für die höchste Verabreichungsmenge (750 PPU kg⁻¹, p<0,05) signifikant war. Die Calcium-Retention gemessen in der Negativkontrolle wurde durch alle Phytasebehandlungen von 181 mg kg⁻¹ 0,75 d⁻¹ auf 240, 245, 266 und 270 mg kg⁻¹ 0,75 d⁻¹ signifikant (p<0,05) verbessert. Die Unterschiede zwischen den Zugabemengen an Phytase waren signifikant (p<0,05) zwischen den höheren Dosierungen (500 und 750 PPU kg⁻¹ Futter) verglichen mit den niedrigeren Dosierungen (125 und 250 PPU kg⁻¹).

Die Ergebnisse zeigen, dass die erfindungsgemäße Phytase eine gute Wirkung auf den Abbau von Phytat im Verdauungstrakt von Schweinen hatte. Bereits niedrige Mengen der erfindungsgemäßen Phytase können die Calcium- und Phosphat-Verdaulichkeit und -Retention in ähnlichem Umfang wie höhere Zugabemengen fördern.

### Beispiel 8: Verbesserung der Verfügbarkeit von Phosphat durch die erfindungsgemäße Phytase (Tyr²⁰⁰-Mutante) bei Masthühnern

Ein Verdauungsversuch wurde mit Masthühnern in 2 Sammelperioden durchgeführt. Es wurde eine Sammelperiode von 4 Tagen nach einer Anpassungsperiode an die Käfige von ebenfalls 4 Tagen durchgeführt. Die Sammelperioden wurden am Ende der Starter-Phase und am Ende der Wachstumsphase durchgeführt. Eine Ration mit reduziertem Gehalt an verdaulichem Phosphat auf der Basis von Mais- und Sojaextraktionsschrot wurde als Negativkontrolle verwendet und mit der erfindungsgemäßen Phytase in Mengen von 125 und 250 PPU kg⁻¹ Futter supplementiert. Zusätzlich wurde eine weitere Behandlung mit empfohlenem Phosphatgehalt ohne Phytasezusatz getestet. Insgesamt 24 männliche Masthühner wurden auf 4 Behandlungsgruppen verteilt. Die Behandlungsgruppen bestanden aus 6 Masthühnern je Sammelzeitraum. Die Behandlungen waren wie folgt:
Negativkontrolle¹ (NC)
NC + Phytase (125 PPU kg⁻¹)
NC + Phytase (250 PPU kg⁻¹)
Positivkontrolle²
   ¹Futter. 53,3-56,5% Maismehl; 37,9-34,5% Sojaextraktionsschrot ; 4,4-4,7 g kg⁻¹ gesamt P; 2,0 g kg⁻¹ Nicht-Phytat P; 6,4-5,9 g kg⁻¹ Ca
   ²Futter. 50,4-54,3% Maismehl; 35,9-35,0% Sojaextraktionsschrot; 7,3-6,4 g kg⁻¹ gesamt P; 4,5-3,5 g kg⁻¹ Nicht-Phytat P; 9,8-7,8 g kg⁻¹ Ca

Die gemessenen Parameter umfassen Phosphatausscheidung und Phosphat-Retention. Die Ergebnisse zeigen, dass beide Zugabemengen von Phytase P (125 und 250 PPU kg⁻¹ Futter) die Phosphat-Retention erhöhten und die Phosphat-Exkretion senkten. Die Messungen am Ende der Startphase zeigten eine signifikante Zunahme der Phosphat-Retention von 58,7% in der Negativkontrolle auf 64,2 und 63,9% für die Phytasebehandlungen (p<0,05). Dies führte zu einer Abnahme der Phosphatausscheidung bei Masthühnern, die Phytase erhalten hatten, die verglichen mit der Negativkontrolle um 11,4 und 12,7% (Tendenz) verringert war. Messungen am Ende der Wachstumsphase zeigten ebenfalls eine Zunahme der Phosphat-Retention von 54,7% in der Negativkontrolle auf 58,2 und 58,9% für die Phytasebehandlungen (Tendenz). Dies führte zu einer Abnahme der Phosphat-Exkretion bei Masthühnern, die Phytase erhalten hatten, die verglichen mit der Negativkontrolle um 11 % und 12,7% signifikant (p<0,05) verringert war.

### Beispiel 9: Backversuch: Vienna Brot

Vienna Brot wurde aus 320 g Teigstücken, erhalten durch Mischen von 1000 g Weizenmehl (Pfälzer Mühlenwerke, Mannheim, Type 550), 30 g Presshefe (Fala GmbH, Deutschland), 20 g Salz, 50 mg Ascorbinsäure und 580 g Wasser gebacken. Nach 2-minütigem Mischen aller Inhaltsstoffe mit langsamer Geschwindigkeit und 6-minütigem Mischen mit Hochgeschwindigkeit (Diosna SP12 Mischer) hatte der Teig eine Temperatur von 27°C und wurde 10 Minuten bei Umgebungstemperatur (22°C) unter einer Tuchabdeckung stehen gelassen. Nach der ersten Teigruhe wurde der Teig in Stücke zu je 320 g (± 1 g Toleranz) aufgeteilt und zu einer runden Form geformt. Danach folgte eine zweite Teigruhe von 20 Minuten. Nach der zweiten Teigruhe wurde der Teig in einer mechanischen Vorrichtung geformt und auf mit Tuch bedeckte Fermentationsbleche gelegt. Der Brotteig wurde dann bei 32°C unter 85% relativer Feuchtigkeit fermentiert (abschließende Reifung) und nach einer Reifungszeit von 70 Minuten gebacken. Der Teig/das Brot wurde in einem mehrschienigen Ofen (Winkler & Wachtel, Deutschland) mit 5-sekündiger Dampfinjektion für 35 Minuten bei 235°C gebacken.

Die verschiedenen Wirkungen der erfindungsgemäßen Phytase (*E*. *coli-*Phytasemutante Tyr²⁰⁰) in den Backexperimenten wurden parallel mit einem Kontrollteig ohne Phytasezusatz verglichen. Das Volumen der Kontrollbrote wurde als 100% genommen.

Das Volumen der Brotleibe wurde durch das Rapssamenverdrängungsverfahren bestimmt. Die Teigrheologie und die Broteigenschaften wurden sensorisch durch einen qualifizierten Anwendungsspezialisten/Testbäcker bewertet und das Durchschnittsvolumen der drei Laibe pro Test wurde gemessen.

Die Ergebnisse des Backversuches sind in der folgenden Tabelle 4 zusammengestellt:

**Tabelle 4**

| **Versuch** | **Units je kg Mehl** | | | | | | | | | | |
|---|---|---|---|---|---|---|---|---|---|---|---|
| | **0** | **110** | **220** | **430** | **870** | **1730** | **3460** | **6930** | **13860** | **27715** | **55430** |
| **Teig nach Anteigen** | | | | | | | | | | | |
| Festigkeit | 4 | 4,5 | 4,5 | 5 | 5 | 5 | 5 | 5 | 6 | 6 | 6 |

| **Teig nach Endgare** | | | | | | | | | | | |
|---|---|---|---|---|---|---|---|---|---|---|---|
| Volumen | 4 | 4 | 4 | 4 | 4 | 4 | 4 | 4 | 4 | 4 | 4 |
| Stabilität | 4 | 4 | 4 | 5 | 5 | 5 | 5 | 5 | 5,5 | 5,5 | 5,5 |

| Beurteilungskriterien: | | | | | | | | | | | |
|---|---|---|---|---|---|---|---|---|---|---|---|
| **Beurteilungskriterien** | **1** | | **2** | **3** | | **4** | **5** | | **6** | **7** | |
| **Teig nach Anteigen** | | | | | | | | | | | |
| Festigkeit | viel weicher | | weicher | etwas weicher | | wie Referenz | etwas fester | | fester | viel fester | |

| **Teig nach Endgare** | | | | | | | | | | | |
|---|---|---|---|---|---|---|---|---|---|---|---|
| Volumen | viel kleiner | | kleiner | etwas kleiner | | wie Referenz | etwas größer | | größer | viel größer | |
| Stabilität | sehr instabil | | instabil | etwas instabil | | wie Referenz | etwas stabiler | | stabiler | viel stabiler | |

### Erläuterung der Beurteilungskriterien:

Festigkeit: Steifheit des Teigs beurteilt durch einen erfahrenen Bäcker.
Volumen: Visuelle Beurteilung des Volumens des Teiglings nach der Endgare.
Stabilität: Test des Gasrückhaltevermögens durch Druckbelastung des Teiglings nach der Endgare und Beurteilung der eingetretenen Volumenverkleinerung.

Die vorstehenden Ergebnisse zeigen, dass die erfindungsgemäße Phytase eine stabilisierende Wirkung auf den Teig hatte.

Die erfindungsgemäße Phytase hatte eine stabilisierende Wirkung auf den Teig.

Die Versuche gemäß den Beispielen 7 bis 9 zeigen, dass die erfindungsgemäße *E. coli*-Phytasemutante Tyr²⁰⁰ sich in ihrer Wirkung von der Wildtypphytase nicht unterscheidet. Die erfindungsgemäße *E. coli*-Phytasemutante Tyr²⁰⁰ zeichnet sich jedoch durch den Vorteil einer höheren Aktivität im Kulturüberstand in der Summe bzw. höhere Sekretionseffizienz verglichen mit dem Wildtyp aus.

### Beispiel 10: Konstruktion des Plasmids pKDa41

Das Plasmid pKDa41 enthält das *E. coli*-Phytase-Gen (WT) unter der Kontrolle des *T*. *reesei cbh*I Promotors und des *cbh*I Terminators. Die Konstruktion ist vergleichbar mit der Konstruktion des Plasmids pKDa4, außer dass die 16 Basenpaare stromaufwärts des Phytase-Startcodons, CCGCGGACTGCGCATC ATG zu CCGCGGACT**AG**GCATC ATG geändert wurden und eine PacI Restriktionsspaltstelle unmittelbar stromabwärts des Stopcodons lokalisiert wurde.

Zur Konstruktion des Plasmids pKDa41 wurde das *E. coli*-Phytase-Gen (WT) aus dem Plasmid pKDa4 mittels PCR amplifiziert. Das PCR-Produkt wurde mit Avrll und PacI gespalten und in die SpeI und PacI Spaltstellen nach dem *T*. *reesei cbh*I Promotor in dem Plasmid pAB489 inseriert. Das entstandene Plasmid hat die Bezeichnung pKDa41 und wurde durch Restriktionsendonucleasen kartiert und die Phytasesequenz durch Sequenzierung bestätigt. Die aus dem Plasmid pKDa41 isolierte Expressionskassette (NotI-Fragment) enthält identisch genetische Materialien, wie die aus dem Plasmid pKDa4. Das Plasmid pKDa41 wurde als Ausgangsmaterial zur Herstellung für diverse Phytasevarianten verwendet sowie als Referenz bei der Untersuchung der Phytase-Expression in *T. reesei* RH3780d.

Das Plasmid pAB489 geht aus dem Plasmid pALK487 (WO 94/28117) durch Einfügen weiterer Schnittstellen (SpeI und PacI) in die SacII Site zwischen dem in pALK487 enthaltenen *cbh*I Promotor und *cbh*I Terminator, sowie des in Beispiel 2 aufgeführten 4,78 kb langen *Ec*oRI/*Spe*I-Fragments des Plasmids pALK424 (WO 93/24621), das den *amdS* Marker und die 3'-flankierenden cbhl-Sequenzen enthält, hervor. Die Anordnung der Elemente ist wie in pKDa4 und erlaubt die direkte Klonierung der Genvarianten in die SpeI und PacI Spaltstellen der Multicloning Site nach dem *T*. *reesei cbh*I Promotor.

### Beispiel 11: Konstruktion der Phytasevarianten-Plasmide

Folgende Phytasevarianten-Plasmide wurden konstruiert:
pPhy-V200L, pPhy-V200P, pPhy-L207F

Zur Herstellung der Phytasevarianten wurden die Mutationen des PhytaseGens mittels der PCR Methode analog dem Prinzip, das in Nucleic Acids Research 1989, 17(2), 723-733 und Nucleic Acids Research 1990, 18(6), 1656 beschrieben ist, durchgeführt. Die Konstruktion sowie die Klonierung der Phytasevarianten-Plasmide sind identisch mit der in Beispiel 10 beschriebenen Herstellung des Plasmids pKDa41. Die Sequenzen der Phytasevarianten wurden durch Sequenzierung bestätigt.

### Beispiel 12: Transformation von T. reesei RH3780d mit pKDa41 bzw. Varianten

Die Transformation von *T*. *reesei* RH3780d mit den aus dem Plasmid pKDa41 aus Beispiel 10 und den Phytasevarianten-Plasmiden aus Beispiel 11 isolierten Expressionskassetten wurde analog zu der Transformation mit den aus den Plasmiden pKDa2 und pKDa4 isolierten Expressionskassetten durchgeführt (Beispiel 3). Die Expressionskassetten aus den Plasmiden pKDa2, pKDa4, pKDa41 und Phytasevarianten-Plasmiden wurden als NotI-Fragmente isoliert.

### Beispiel 13: Produktion von E. coli-Phytase durch pKDa41- und Phytasevarianten-Expressionskassetten in Schüttelkolben

Die Transformanten wurden wie in Beispiel 4 beschrieben angezüchtet und die Phytase in den Kulturfiltraten für weitere Untersuchungen herangezogen.

**Tabelle 5: Produktion von E. coli-Phytase durch Transformanten, die entweder die pKDa41- oder Phytasevarianten-Expressionskassetten enthalten.**

| **Stamm** | **SDS-PAGE** | **Phytase-Kassette Kopieanzahl** | **Phytase PPU g⁻¹** | **Expressionskassette** |
|---|---|---|---|---|
| RH31551 | CBHI⁻ | eine Kopie | 82 | pKDa41 |
| RH31549 | CBHI⁻ | eine Kopie | 76 | pKDa41 |
| | | | | |
| RH31565 | CBHI⁻ | eine Kopie | 225 | pPhy-V200L |
| RH31567 | CBHI⁻ | eine Kopie | 230 | pPhy-V200L |
| RH31570 | CBHI⁻ | eine Kopie | 291 | pPhy-V200P |
| RH31571 | CBHI⁻ | eine Kopie | 295 | pPhy-V200P |
| RH31559 | CBHI⁻ | eine Kopie | 114 | pPhy-L207F |
| RH31563 | CBHI⁻ | eine Kopie | 112 | pPhy-L207F |
| RH3780d | CBHI⁺ | | 0,7 | |

## Patentansprüche

1. Rekombinantes DNA-Molekül, das nach Expression in einer prokaryotischen oder eukaryotischen Wirtszelle ein Polypeptid mit Phytaseaktivität codiert, wobei das rekombinante DNA-Molekül eine DNA-Sequenz, ausgewählt aus
a) DNA-Sequenzen, die durch Variationen der reifen Wildtyp-*E*. *coli-*Phytasesequenz erhalten worden sind, wobei die DNA-Sequenzen mindestens eine Mutation aufweisen, die ausgewählt ist aus der Gruppe Val 200 → Leu, Val 200 → Ile, Val 200 → Pro, Val 200 → Tyr, Leu 207 → Tyr und Leu 207 → Phe,
b) DNA-Sequenzen, die aufgrund der Degeneriertheit des genetischen Codes mit den Sequenzen gemäß a) verwandt sind,
umfasst, wobei das rekombinante DNA-Molekül bei Expression in einer geeigneten Wirtszelle mit einer erhöhten Aktivität des so codierten Proteins im Kulturüberstand einhergeht.

2. Rekombinantes DNA-Molekül nach Anspruch 1, **dadurch gekennzeichnet, dass** es die Sequenz SEQ ID NO: 1 besitzt.

3. Rekombinantes DNA-Molekül nach einem der Ansprüche 1 bis 2, **dadurch gekennzeichnet, dass** es weiterhin eine aus dem Gen von *Aspergillus niger*-Phytase abgeleitete Signalsequenz mit der Sequenz SEQ ID NO: 3 umfasst.

4. Polypeptid, das Phytaseaktivität besitzt und von einem rekombinanten DNA-Molekül nach einem der Ansprüche 1 bis 3 codiert wird oder durch Expression einer damit transformierten Wirtszelle erhalten wird.

5. Polypeptid nach Anspruch 4, **dadurch gekennzeichnet, dass** es die Sequenz SEQ ID NO: 2 besitzt.

6. DNA-Konstrukt mit der Fähigkeit, nach dem Einführen in eine geeignete Wirtszelle die Expression eines mutierten Phytasegens in einem Wirt zu steuern, **dadurch gekennzeichnet, dass** es gegebenenfalls einen Promotor, gegebenenfalls Signal- und Markersequenzen, eine DNA-Sequenz nach einem der Ansprüche 1 bis 2, einen Terminator und gegebenenfalls 5'- und 3'-flankierende Sequenzen enthält.

7. DNA-Konstrukt nach Anspruch 6, **dadurch gekennzeichnet, dass** es sich bei dem Promotor um den Cellobiohydrolase-I-, den Cellobiohydrolase-II-, den Amylase-, den Glucoamylase-, den Xylanase- oder den Enolase-Promotor handelt.

8. DNA-Konstrukt nach Anspruch 6, **dadurch gekennzeichnet, dass** es sich bei der Signalsequenz um eine gegebenenfalls modifizierte Phytase-Signalsequenz aus *Aspergillus niger,* bevorzugt die Sequenz SEQ ID NO: 3, handelt.

9. Vektor mit der Fähigkeit, eine Wirtszelle zu transformieren, **dadurch gekennzeichnet, dass** der Vektor ein Konstrukt nach Anspruch 6 enthält.

10. Vektor nach Anspruch 9, **dadurch gekennzeichnet, dass** er das Plasmid Da2pUC3, hinterlegt unter der Hinterlegungsnummer DSM 16396, ist.

11. Transformierte Wirtszelle, ausgewählt aus Pilz, Hefe, Bakterien und Säugerzellen, enthaltend ein rekombinantes DNA-Molekül nach einem der Ansprüche 1 bis 3 und ausgestattet mit der Fähigkeit, ein Polypeptid mit Phytaseaktivität zu exprimieren.

12. Transformierte Wirtszelle nach Anspruch 11, **dadurch gekennzeichnet, dass** sie der Gattung Aspergillus, Rhizopus, Trichoderma, Neurospora, Mucor oder Penicillium angehört.

13. Verfahren zur Produktion von Phytase, **dadurch gekennzeichnet, dass** eine transformierte Wirtszelle nach Anspruch 11 oder 12 unter Bedingungen gezüchtet wird, die der Bildung von Phytase förderlich sind und die so produzierte Phytase isoliert wird.

14. Zusammensetzung, umfassend ein Polypeptid nach Anspruch 4 oder 5, gegebenenfalls zusammen mit weiteren Hilfs- und/oder Wirkstoffen.

15. Zusammensetzung nach Anspruch 14, **dadurch gekennzeichnet, dass** es eine Nahrungsmittel- oder Futtermittelzusammensetzung ist.

16. Zusammensetzung nach Anspruch 14, **dadurch gekennzeichnet, dass** die Zusammensetzung ein Backmittel ist.

17. Verwendung eines Polypeptids nach Anspruch 4 oder 5 zur Herstellung eines Präparats zur Verbesserung der Phosphatverwertung aus der Nahrung bei Tieren und Menschen.

18. Verwendung eines Polypeptids nach Anspruch 4 oder 5 zur Verbesserung der rheologischen Eigenschaften von Teigen zur Herstellung von Backwaren.

## Claims

1. A recombinant DNA molecule which, upon expression in a prokaryotic or eukaryotic host cell, encodes a polypeptide having phytase activity, wherein the recombinant DNA molecule comprises a DNA sequence selected from
a) DNA sequences which have been obtained by variations of the mature wild-type *E.coli* phytase sequence, wherein the DNA sequences exhibit at least one mutation selected from the group Val 200 -> Leu, Val 200 -> Ile, Val 200 -> Pro, Val 200 -> Tyr, Leu 207 -> Tyr, Leu 207 -> Phe,
b) DNA sequences which are related to the sequences according to a) due to the degeneracy of the genetic code,
wherein the recombinant DNA molecule is, upon expression in a suitable host cell, associated with an increased activity of the thus encoded protein in the culture supernatant.

2. The recombinant DNA molecule according to claim 1, **characterized in that** it has the sequence of SEQ ID NO: 1.

3. The recombinant DNA molecule according to one of claims 1 to 2, **characterized in that** it further comprises a signal sequence with the sequence of SEQ ID NO: 3 derived from the gene of the phytase from *Aspergillus niger.*

4. A polypeptide which has phytase activity and is encoded by a recombinant DNA molecule according to one of claims 1 to 3 or is obtained by expression of a host cell transformed therewith.

5. The polypeptide according to claim 4, **characterized in that** it has the sequence of SEQ ID NO: 2.

6. A DNA construct having the capability to control the expression of a mutated phytase gene in a host upon introduction in a suitable host cell, **characterized in that** it contains optionally a promoter, optionally signal- and marker sequences, a DNA sequence according to one of claims 1 to 2, a terminator and optionally 5'- and 3'-flanking sequences.

7. The DNA construct according to claim 6, **characterized in that** the promoter is the cellobiohydrolase I, the cellobiohydrolase II, the amylase, the glucoamylase, the xylanase or the enolase promoter.

8. The DNA construct according to claim 6, **characterized in that** the signal sequence is an optionally modified phytase signal sequence from *Aspergillus niger,* preferably the sequence of SEQ ID NO: 3.

9. A vector having the capability to transform a host cell, **characterized in that** the vector contains a construct according to claim 6.

10. The vector according to claim 9, **characterized in that** it is the plasmid Da2pUC3, deposited under the accession number DSM 16396.

11. A transformed host cell, selected from fungus, yeast, bacteria and mammalian cells, containing a recombinant DNA molecule according to one of claims 1 to 3 and being provided with the capability to express a polypeptide having phytase activity.

12. The transformed host cell according to claim 11, **characterized in that** it belongs to the genus Aspergillus, Rhizopus, Trichoderma, Neurospora, Mucor or Penicillium.

13. A method for producing phytase, **characterized in that** a transformed host cell according to claim 11 or 12 is grown under conditions conductive to the formation of phytase and the thus produced phytase is isolated.

14. A composition comprising a polypeptide according to claim 4 or 5, optionally together with further auxiliary and/or active agents.

15. The composition according to claim 14, **characterized in that** it is a food or feed composition.

16. The composition according to claim 14, **characterized in that** the composition is a baking aid.

17. Use of a polypeptide according to claims 4 or 5 for the manufacture of a preparation for ameliorating the phosphate utilization from nutrition in animals and humans.

18. Use of a polypeptide according to claim 4 or 5 for ameliorating the rheological properties of dough for the production of bakery products.

## Revendications

1. Molécule d'ADN recombinante qui, après expression dans une cellule hôte procaryote ou eucaryote, code un polypeptide ayant une activité de phytase, la molécule d'ADN recombinante comprenant une séquence d'ADN choisie parmi
a) les séquences d'ADN qui ont été obtenues par variations de la séquence de phytase mature de *E*. *coli* de type sauvage, les séquences d'ADN présentant au moins une mutation qui est choisie dans le groupe constitué par Val 200 → Leu, Val 200 → Ile, Val 200 → Pro, Val 200 → Tyr, Leu 207 → Tyr et Leu 207 → Phe,
b) les séquences d'ADN qui sont apparentées aux séquences selon le point a) compte tenu de la dégénérescence du code génétique,
la molécule d'ADN recombinante induisant, lors de l'expression dans une cellule hôte appropriée, une activité accrue de la protéine ainsi codée dans le surnageant de culture.

2. Molécule d'ADN recombinante selon la revendication 1, **caractérisée en ce qu'**elle possède la séquence SEQ ID N°1.

3. Molécule d'ADN recombinante selon l'une des revendications 1 à 2, **caractérisée en ce qu'**elle comprend en outre une séquence signal dérivée du gène de la phytase d'*Aspergillus niger* ayant la séquence SEQ ID N°3.

4. Polypeptide, qui possède une activité de phytase et est codé par une molécule d'ADN recombinante selon l'une des revendications 1 à 3 ou qui est obtenu par l'expression d'une cellule hôte ainsi transformée.

5. Polypeptide selon la revendication 4, **caractérisé en ce qu'**il possède la séquence SEQ ID N°2.

6. Hybride d'ADN ayant la capacité de réguler l'expression d'un gène de phytase muté dans un hôte après l'introduction dans une cellule hôte appropriée, **caractérisé en ce qu'**il comprend le cas échéant un promoteur, le cas échéant des séquences signal et marqueur, une séquence d'ADN selon l'une des revendications 1 à 2, un terminateur et le cas échéant des séquences flanquantes en 5' et 3'.

7. Hybride d'ADN selon la revendication 6, **caractérisé en ce que** le promoteur est un promoteur de cellobiohydrolase-I, de cellobiohydrolase-II, d'amylase, de glucoamylase, de xylanase ou d'énolase.

8. Hybride d'ADN selon la revendication 6, **caractérisé en ce que** la séquence signal est une séquence signal de phytase le cas échéant modifiée provenant de *Aspergillus niger,* de préférence la séquence SEQ ID N°3.

9. Vecteur ayant la capacité de transformer une cellule hôte, **caractérisé en ce que** le vecteur comprend un hybride selon la revendication 6.

10. Vecteur selon la revendication 9, **caractérisé en ce qu'**il est le plasmide Da2pUC3, enregistré sous le numéro d'enregistrement DSM 16396.

11. Cellule hôte transformée choisie parmi le champignon, la levure, les bactéries et les cellules de mammifères, contenant une molécule d'ADN recombinante selon l'une des revendications 1 à 3 et dotée de la capacité d'exprimer un polypeptide ayant une activité de phytase.

12. Cellule hôte transformée selon la revendication 11, **caractérisée en ce qu'**elle appartient au genre *Aspergillus, Rhizopus, Trichoderma, Neurospora, Mucor* et *Penicillium.*

13. Procédé de production de phytase, caractérisé en qu'une cellule hôte transformée selon la revendication 11 ou 12 est cultivée dans des conditions qui sont favorables à la formation de phytase et en ce que la phytase ainsi produite est isolée.

14. Composition comprenant un polypeptide selon la revendication 4 ou 5, le cas échéant conjointement avec d'autres agents auxiliaires ou principes actifs.

15. Composition selon la revendication 14, **caractérisée en ce que** c'est une composition de produit alimentaire ou de nourriture animale.

16. Composition selon la revendication 14, **caractérisée en ce que** la composition est un améliorant pour les produits boulangers et pâtissiers.

17. Utilisation d'un polypeptide selon la revendication 4 ou 5 pour la production d'une préparation destinée à améliorer l'absorption du phosphate à partir des aliments chez les animaux et l'homme.

18. Utilisation d'un polypeptide selon la revendication 4 ou 5 pour l'amélioration des propriétés rhéologiques des pâtes destinées à la préparation des produits boulangers et pâtissiers.


### SEQUENZPROTOKOLL

<110> AB ENZYMES GMBH
<120> POLYPEPTID MIT PHYTASEAKTIVITÄT UND DIESES CODIERENDE NUCLEOTIDSEQUENZ
<130> 14052WO
<140>
   <141>
<160> 4
<170> PatentIn Ver. 2.1
<210> 1
   <211> 1230
   <212> DNA
   <213> Künstliche Sequenz
<220>
   <223> Beschreibung der künstlichen Sequenz:Tyr200-Mutante der reifen E. coli-Wildtyp Phytase
<220>
   <221> CDS
   <222> (1)..(1230)
<400> 1
   cag agc gag ccc gag ctg aag ctg gag tcg gtc gtg atc gtc agc cgc    48
   Gln Ser Glu Pro Glu Leu Lys Leu Glu Ser Val Val Ile Val Ser Arg
    1               5                  10                  15
   cac ggc gtg cgt gct cct acc aag gcc acg cag ctg atg cag gac gtc    96
   His Gly Val Arg Ala Pro Thr Lys Ala Thr Gln Leu Met Gln Asp Val
               20                  25                  30
   acc cct gac gcc tgg ccc acc tgg ccc gtc aag ctt ggc tgg ctg act   144
   Thr Pro Asp Ala Trp Pro Thr Trp Pro Val Lys Leu Gly Trp Leu Thr
           35                  40                  45
   cct cgc ggc ggt gag ctc atc gcc tac ctc gga cac tac caa cgc cag   192
   Pro Arg Gly Gly Glu Leu Ile Ala Tyr Leu Gly His Tyr Gln Arg Gln
       50                  55                  60
   cgt ctg gtt gcc gac gga ctc ctg gct aag aag gga tgc ccg cag tct   240
   Arg Leu Val Ala Asp Gly Leu Leu Ala Lys Lys Gly Cys Pro Gln Ser
   65                  70                  75                  80
   ggc cag gtc gcg att atc gcc gat gtc gac gag cgt acc cgt aag acc   288
   Gly Gln Val Ala Ile Ile Ala Asp Val Asp Glu Arg Thr Arg Lys Thr
                   85                  90                  95
   ggc gaa gcc ttc gct gcc ggc ctc gct cct gac tgt gcc atc acg gtc   336
   Gly Glu Ala Phe Ala Ala Gly Leu Ala Pro Asp Cys Ala Ile Thr Val
               100                 105                 110
   cac acc cag gca gac acg tcc agc ccc gat ccg ctg ttt aac cct ctc    384
   His Thr Gln Ala Asp Thr Ser Ser Pro Asp Pro Leu Phe Asn Pro Leu
           115                 120                 125
   aag act ggc gtc tgc caa ctg gat aac gcc aac gtg acc gac gcc atc    432
   Lys Thr Gly Val Cys Gln Leu Asp Asn Ala Asn Val Thr Asp Ala Ile
       130                 135                 140
   ctc agc agg gct gga ggt tcc atc gcc gac ttc acc ggc cat cgg cag    480
   Leu Ser Arg Ala Gly Gly Ser Ile Ala Asp Phe Thr Gly His Arg Gln
   145                 150                 155                 160
   acg gcg ttc cgc gag ctg gag cgg gtc ctt aat ttt ccc cag tcg aac    528
   Thr Ala Phe Arg Glu Leu Glu Arg Val Leu Asn Phe Pro Gln Ser Asn
                   165                 170                 175
   ctg tgc ctc aag cgt gag aag cag gac gag agc tgt tcc ctg acc cag    576
   Leu Cys Leu Lys Arg Glu Lys Gln Asp Glu Ser Cys Ser Leu Thr Gln
               180                 185                 190
   gca ctc ccg tcg gaa ctc aag tac agc gcc gac aac gtc tcc ctt acc    624
   Ala Leu Pro Ser Glu Leu Lys Tyr Ser Ala Asp Asn Val Ser Leu Thr
           195                 200                 205
   ggt gcc gtt agc ctc gct tcc atg ctg acg gag atc ttc ctc ctg cag    672
   Gly Ala Val Ser Leu Ala Ser Met Leu Thr Glu Ile Phe Leu Leu Gln
       210                 215                 220
   caa gcg cag gga atg ccc gag cct ggg tgg ggc cgc att acc gat tct    720
   Gln Ala Gln Gly Met Pro Glu Pro Gly Trp Gly Arg Ile Thr Asp Ser
   225                 230                 235                 240
   cac cag tgg aac acc ctg ctc tcg ctt cac aac gcc cag ttc tat ctg    768
   His Gln Trp Asn Thr Leu Leu Ser Leu His Asn Ala Gln Phe Tyr Leu
                   245                 250                 255
   ctc caa cgc acg ccc gag gtt gcc cgc agc cgc gcc acc ccg ctg ctc    816
   Leu Gln Arg Thr Pro Glu Val Ala Arg Ser Arg Ala Thr Pro Leu Leu
               260                 265                 270
   gac ctc atc aag act gcg ctg acg ccc cac cct ccg cag aag cag gct    864
   Asp Leu Ile Lys Thr Ala Leu Thr Pro His Pro Pro Gln Lys Gln Ala
           275                 280                 285
   tac ggt gtc acc ctc ccc act tcc gtc ctg ttt atc gcc ggt cac gac    912
   Tyr Gly Val Thr Leu Pro Thr Ser Val Leu Phe Ile Ala Gly His Asp
       290                 295                 300
   acc aac ctg gcc aat ctc ggc ggc gct ctg gag ctc aac tgg acg ctt    960
   Thr Asn Leu Ala Asn Leu Gly Gly Ala Leu Glu Leu Asn Trp Thr Leu
   305                 310                 315                 320
   ccc gga cag ccg gat aac act ccc cct ggc ggt gag ctg gtg ttc gaa    1008
   Pro Gly Gln Pro Asp Asn Thr Pro Pro Gly Gly Glu Leu Val Phe Glu
                   325                 330                 335
   cgc tgg cgt cgg ctc agc gac aac tcc cag tgg att cag gtt tcg ctg     1056
   Arg Trp Arg Arg Leu Ser Asp Asn Ser Gln Trp Ile Gln Val Ser Leu
               340                 345                 350
   gtc ttc cag acc ctg cag cag atg cgc gac aaa acg ccc ctg tcc ctc     1104
   Val Phe Gln Thr Leu Gln Gln Met Arg Asp Lys Thr Pro Leu Ser Leu
           355                 360                 365
   aat acc cct ccc ggc gag gtc aag ctg acc ctg gca ggc tgt gaa gag     1152
   Asn Thr Pro Pro Gly Glu Val Lys Leu Thr Leu Ala Gly Cys Glu Glu
       370                 375                 380
   cgc aac gcc cag ggc atg tgc tct ctc gct ggc ttt acg caa atc gtg     1200
   Arg Asn Ala Gln Gly Met Cys Ser Leu Ala Gly Phe Thr Gln Ile Val
   385                 390                 395                 400
   aac gag gcc cgc atc ccc gct tgc tct ctg                             1230
   Asn Glu Ala Arg Ile Pro Ala Cys Ser Leu
                   405                 410
<210> 2
   <211> 410
   <212> PRT
   <213> Künstliche Sequenz
<223> Beschreibung der künstlichen Sequenz:Tyr200-Mutante der reifen E. coli-Wildtyp Phytase
<400> 2
   Gln Ser Glu Pro Glu Leu Lys Leu Glu Ser Val Val Ile Val Ser Arg
   1               5                   10                  15
   His Gly Val Arg Ala Pro Thr Lys Ala Thr Gln Leu Met Gln Asp Val
               20                  25                  30
   Thr Pro Asp Ala Trp Pro Thr Trp Pro Val Lys Leu Gly Trp Leu Thr
           35                  40                  45
   Pro Arg Gly Gly Glu Leu Ile Ala Tyr Leu Gly His Tyr Gln Arg Gln
       50                  55                  60
   Arg Leu Val Ala Asp Gly Leu Leu Ala Lys Lys Gly Cys Pro Gln Ser
   65                  70                  75                  80
   Gly Gln Val Ala Ile Ile Ala Asp Val Asp Glu Arg Thr Arg Lys Thr
                   85                  90                  95
   Gly Glu Ala Phe Ala Ala Gly Leu Ala Pro Asp Cys Ala Ile Thr Val
               100                 105                 110
   His Thr Gln Ala Asp Thr Ser Ser Pro Asp Pro Leu Phe Asn Pro Leu
           115                 120                 125
   Lys Thr Gly Val Cys Gln Leu Asp Asn Ala Asn Val Thr Asp Ala Ile
       130                 135                 140
   Leu Ser Arg Ala Gly Gly Ser Ile Ala Asp Phe Thr Gly His Arg Gln
   145                 150                 155                 160
   Thr Ala Phe Arg Glu Leu Glu Arg Val Leu Asn Phe Pro Gln Ser Asn
                   165                 170                 175
   Leu Cys Leu Lys Arg Glu Lys Gln Asp Glu Ser Cys Ser Leu Thr Gln
               180                 185                 190
   Ala Leu Pro Ser Glu Leu Lys Tyr Ser Ala Asp Asn Val Ser Leu Thr
           195                 200                 205
   Gly Ala Val Ser Leu Ala Ser Met Leu Thr Glu Ile Phe Leu Leu Gln
       210                 215                 220
   Gln Ala Gln Gly Met Pro Glu Pro Gly Trp Gly Arg Ile Thr Asp Ser
   225                 230                 235                 240
   His Gln Trp Asn Thr Leu Leu Ser Leu His Asn Ala Gln Phe Tyr Leu
                   245                 250                 255
   Leu Gln Arg Thr Pro Glu Val Ala Arg Ser Arg Ala Thr Pro Leu Leu
               260                 265                 270
   Asp Leu Ile Lys Thr Ala Leu Thr Pro His Pro Pro Gln Lys Gln Ala
           275                 280                 285
   Tyr Gly Val Thr Leu Pro Thr Ser Val Leu Phe Ile Ala Gly His Asp
       290                 295                 300
   Thr Asn Leu Ala Asn Leu Gly Gly Ala Leu Glu Leu Asn Trp Thr Leu
   305                 310                 315                 320
   Pro Gly Gln Pro Asp Asn Thr Pro Pro Gly Gly Glu Leu Val Phe Glu
                   325                 330                 335
   Arg Trp Arg Arg Leu Ser Asp Asn Ser Gln Trp Ile Gln Val Ser Leu
               340                 345                 350
   Val Phe Gln Thr Leu Gln Gln Met Arg Asp Lys Thr Pro Leu Ser Leu
           355                 360                 365
   Asn Thr Pro Pro Gly Glu Val Lys Leu Thr Leu Ala Gly Cys Glu Glu
       370                 375                 380
   Arg Asn Ala Gln Gly Met Cys Ser Leu Ala Gly Phe Thr Gln Ile Val
   385                 390                 395                 400
   Asn Glu Ala Arg Ile Pro Ala Cys Ser Leu
                   405                 410
<210> 3
   <211> 54
   <212> DNA
   <213> Künstliche Sequenz
<220>
   <223> Beschreibung der künstlichen Sequenz:modifizierte A. niger Phytase Signalsequenz (ohne Intron)
<220>
   <221> CDS
   <222> (1)..(54)
<400> 3
   atg ggc gtc tct gct gtt cta ctt cct ttg tat ctc ctg tct gga gtc    48
   Met Gly Val Ser Ala Val Leu Leu Pro Leu Tyr Leu Leu Ser Gly Val
     1               5                  10                  15
   acc tcc                                                            54
   Thr Ser
<210> 4
   <211> 18
   <212> PRT
   <213> Künstliche Sequenz
<223> Beschreibung der künstlichen Sequenz:modifizierte A. niger Phytase Signalsequenz (ohne Intron)
<400> 4
   Met Gly Val Ser Ala Val Leu Leu Pro Leu Tyr Leu Leu Ser Gly Val
     1               5                  10                  15
   Thr Ser